# EUROPEAN PATENT SPECIFICATION

(11) **EP 3 923 940 B1**
(45) Date of publication and mention of the grant of the patent: **03.12.2025**
(21) Application number: 20707826.2
(22) Date of filing: 10.02.2020
(51) Int. Cl.: A61K 31/497, A61K 45/06, A61P 35/00

(54) **PHARMACEUTICAL COMBINATION COMPRISING TNO155 AND A PD-1 INHIBITOR**
PHARMAZEUTISCHE KOMBINATION MIT TNO155 UND EINEM PD-1-INHIBITOR
COMBINAISON PHARMACEUTIQUE COMPRENANT DU TNO155 ET UN INHIBITEUR DE PD-1

(30) Priority: 12.02.2019 US 201962804707 P
(43) Date of publication of application: 22.12.2021
(73) Proprietor: Novartis AG, 4056 Basel (CH)
(72) Inventor: CHEN, Ying-nan Pan, Wilmington, Delaware 19808 (US); HAO, Huaixiang, Cambridge, Massachusetts 02139 (US); LIU, Chen, Cambridge, Massachusetts 02139 (US); MOHSENI, Morvarid, Cambridge, Massachusetts 02139 (US); HASTINGS, William D., Cambridge, MA 02139 (US); GOLDONI, Silvia, Winchester, MA 01890 (US)
(74) Representative: Strang, Andrea Josephine
(86) International application number: PCT/IB2020/051030
(87) International publication number: WO 2020/165733

(56) References cited:
- WO-A1-2018/130928
- DEMPKE WOLFRAM C M ET AL: "Targeting SHP-1, 2 and SHIP Pathways: A Novel Strategy for Cancer Treatment?", ONCOLOGY (BASEL), vol. 95, no. 5, 2018, pages 257 - 269, XP009520304
- GIOPANOU IOANNA ET AL: "RAS and BRAF in the foreground for non-small cell lung cancer and colorectal cancer: Similarities and main differences for prognosis and therapies", CRITICAL REVIEWS IN ONCOLOGY/HEMATOLOGY, ELSEVIER, AMSTERDAM, NL, vol. 146, 17 December 2019 (2019-12-17), XP086068685, ISSN: 1040-8428, [retrieved on 20191217], DOI: 10.1016/J.CRITREVONC.2019.102859

## Description

### SEQUENCE LISTING

The instant application contains a Sequence Listing which has been submitted electronically in ASCII format. Said ASCII copy, created on January 17, 2020, is named PAT058373-WO-PCT_SL.txt and is 40,129 bytes in size.

### FIELD OF THE INVENTION

The present invention relates to a pharmaceutical combination comprising TNO155 and a PD-1 inhibitor; such combinations for use in the treatment of cancers.

### BACKGROUND OF THE INVENTION

TNO155 is an orally bioavailable, allosteric inhibitor of Src homology-2 domain containing protein tyrosine phsophatase-2 (SHP2, encoded by the *PTPN11* gene), which transduces signals from activated receptor tyrosine kinases (RTKs) to downstream pathways, including the mitogen-activated protein kinase (MAPK) pathway. SHP2 has also been implicated in immune checkpoint and cytokine receptor signaling. TNO155 has demonstrated efficacy in a wide range of RTK-dependent human cancer cell lines and *in vivo* tumor xenografts.

The Programmed Death 1 (PD-1) protein is an inhibitory member of the extended CD28/CTLA-4 family of T cell regulators. Two ligands for PD-1 have been identified, PD-L1 (B7-H1) and PD-L2 (B7-DC), that have been shown to downregulate T cell activation upon binding to PD-1. PD-L1 is abundant in a variety of human cancers.

PD-1 is known as an immunoinhibitory protein that negatively regulates TCR signals. The interaction between PD-1 and PD-L1 can act as an immune checkpoint, which can lead to, for example, a decrease in tumor infiltrating lymphocytes, a decrease in T-cell receptor mediated proliferation, and/or immune evasion by cancerous cells. Immune suppression can be reversed by inhibiting the local interaction of PD-1 with PD-L1 or PD-L2; the effect is additive when the interaction of PD-1 with PD-L2 is blocked as well.

Given the importance of immune checkpoint pathways in regulating an immune response, the need exists for developing novel combination therapies that activate the immune system.

WO2018/130928 discusses the use of the SHP2 inhibitor TNO155 in the treatment of cancer in combination with an ALK inhibitor.

### SUMMARY OF THE INVENTION

The present invention provides for a pharmaceutical combination comprising:
(a) (3S,4S)-8-(6-amino-5-((2-amino-3-chloropyridin-4-yl)thio)pyrazin-2-yl)-3-methyl-2-oxa-8-azaspiro[4.5]decan-4-amine (TNO155), or a pharmaceutically acceptable salt thereof, having the structure: 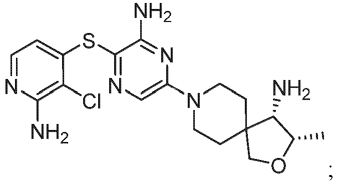 and
(b) an immune checkpoint inhibitor.

In an embodiment, the immune checkpoint inhibitor is a PD-1 inhibitor.

In a further embodiment, the PD-1 inhibitor is chosen from PDR001 (spartalizumab; Novartis), Nivolumab (Bristol-Myers Squibb), Pembrolizumab (Merck & Co), Pidilizumab (CureTech), MEDI0680 (Medimmune), REGN2810 (Regeneron), TSR-042 (Tesaro), PF-06801591 (Pfizer), BGB-A317 (Beigene), BGB-108 (Beigene), INCSHR1210 (Incyte), or AMP-224 (Amplimmune).

In a further embodiment, the PD-1 inhibitor is PDR001 (spartalizumab).

In a further embodiment, the PD-1 inhibitor is administered at a dose of about 300-400 mg.

In a further embodiment, the PD-1 inhibitor is administered once every 3 weeks or once every 4 weeks.

In another embodiment, the PD-1 inhibitor is administered at a dose of about 300 mg once every 3 weeks.

In another embodiment, the PD-1 inhibitor is administered at a dose of about 400 mg once every 4 weeks.

In a further embodiment is provided a pharmaceutical combination comprising:
(a) (3S,4S)-8-(6-amino-5-((2-amino-3-chloropyridin-4-yl)thio)pyrazin-2-yl)-3-methyl-2-oxa-8-azaspiro[4.5]decan-4-amine (TNO155), or a pharmaceutically acceptable salt thereof, having the structure: 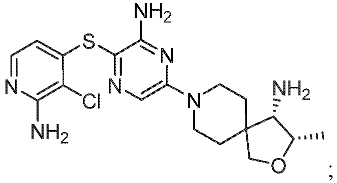 and
(b) PDR001 (spartalizumab).

Combinations of TNO155, or a pharmaceutically acceptable salt thereof, and an immune checkpoint inhibitior (e.g. a PD-1 inhibitor), will also be referred to herein as a "combination of the invention".

In another embodiment of the combination of the invention, TNO155 or a pharmaceutically acceptable salt thereof and a PD-1 inhibitor are in separate formulations.

In another embodiment, the combination of the invention is for simultaneous or sequential (in any order) administration.

In another embodiment is (3S,4S)-8-(6-amino-5-((2-amino-3-chloropyridin-4-yl)thio)pyrazin-2-yl)-3-methyl-2-oxa-8-azaspiro[4.5]decan-4-amine (TNO155), or a pharmaceutically acceptable salt thereof, for use in the treatment of cancer, wherein the treatment further comprises administration of an immune checkpoint inhibitor.

In another embodiment is an immune checkpoint inhibitor for use in the treatment of cance,r wherein the treatment further comprises administration of (3S,4S)-8-(6-amino-5-((2-amino-3-chloropyridin-4-yl)thio)pyrazin-2-yl)-3-methyl-2-oxa-8-azaspiro[4.5]decan-4-amine (TNO155), or a pharmaceutically acceptable salt thereof.

In an embodiment, the cancer is selected from: esophageal or head and neck squamous cell carcinoma; colorectal, ovarian, pancreatic or non-small cell lung cancer; and renal cell carcinoma.

In a further embodiment, the cancer is colorectal cancer.

In a further embodiment, the cancer is non-small cell lung cancer.

In a further embodiment, the cancer is head and neck squamous cell carcinoma.

In another embodiment is a pharmaceutical composition comprising the combination of the invention.

In a further embodiment, the pharmaceutical composition further comprises one or more pharmaceutically acceptable excipients as described herein.

### BRIEF DESCRIPTION OF THE DRAWINGS

Figure 1: Anti-tumor activity of TNO155 as a single agent and in combination with mouse anti-PD1 antibody in a syngeneic immune-competent mouse xenograft model
Figure 2: Immunophenotyping by flow cytometry of MC38 xenograft tumors 7 days post-treatment with TNO155 and combination of TNO155 and mouse anti-PD1 antibody.

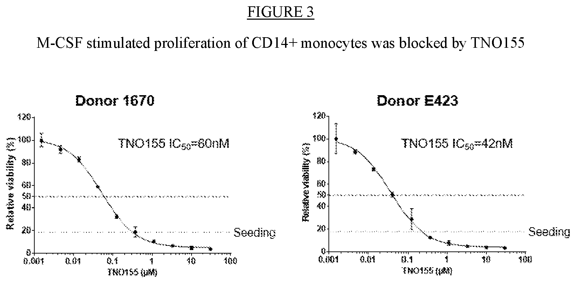

Figure 3: M-CSF stimulated proliferation of CD14+ monocytes was blocked by TNO155.

### Definitions

The general terms used hereinbefore and hereinafter preferably have within the context of this disclosure the following meanings, unless otherwise indicated, where more general terms whereever used may, independently of each other, be replaced by more specific definitions or remain, thus defining more detailed embodiments of the invention:

The term "subject" or "patient" as used herein is intended to include animals, which are capable of suffering from or afflicted with a cancer or any disorder involving, directly or indirectly, a cancer. Examples of subjects include mammals, e.g., humans, apes, monkeys, dogs, cows, horses, pigs, sheep, goats, cats, mice, rabbits, rats, and transgenic non-human animals. In an embodiment, the subject is a human, e.g., a human suffering from, at risk of suffering from, or potentially capable of suffering from cancers.

The term "treating" or "treatment" as used herein comprises a treatment relieving, reducing or alleviating at least one symptom in a subject or effecting a delay of progression of a disease. For example, treatment can be the diminishment of one or several symptoms of a disorder or partial or complete eradication of a disorder, such as cancer. Within the meaning of the present disclosure, the term "treat" also denotes to arrest, delay the onset (i.e., the period prior to clinical manifestation of a disease) and/or reduce the risk of developing or worsening a disease.

The terms "comprising" and "including" are used herein in their open-ended and non-limiting sense unless otherwise noted.

The terms "a" and "an" and "the" and similar references in the context of describing the invention (especially in the context of the following claims) are to be construed to cover both the singular and the plural, unless otherwise indicated herein or clearly contradicted by context. Where the plural form is used for compounds, salts, and the like, this is taken to mean also a single compound, salt, or the like.

The term "combination therapy" or "in combination with" refers to the administration of two or more therapeutic agents to treat a condition or disorder described in the present disclosure (e.g., cancer). Such administration encompasses co-administration of these therapeutic agents in a substantially simultaneous manner, such as in a single capsule having a fixed ratio of active ingredients. Alternatively, such administration encompasses co-administration in multiple, or in separate containers (*e.g.,* capsules, powders, and liquids) for each active ingredient. Powders and/or liquids may be reconstituted or diluted to a desired dose prior to administration. In addition, such administration also encompasses use of each type of therapeutic agent in a sequential manner, either at approximately the same time or at different times. In either case, the treatment regimen will provide beneficial effects of the drug combination in treating the conditions or disorders described herein.

The combination therapy can provide "synergy" and prove "synergistic", i.e., the effect achieved when the active ingredients used together is greater than the sum of the effects that results from using the compounds separately. A synergistic effect can be attained when the active ingredients are: (1) co-formulated and administered or delivered simultaneously in a combined, unit dosage formulation; (2) delivered by alternation or in parallel as separate formulations; or (3) by some other regimen. When delivered in alternation therapy, a synergistic effect can be attained when the compounds are administered or delivered sequentially, *e.g.,* by different injections in separate syringes. In general, during alternation therapy, an effective dosage of each active ingredient is administered sequentially, *i.e.,* serially, whereas in combination therapy, effective dosages of two or more active ingredients are administered together. Synergistic effect, as used herein, refers to action of two therapeutic agents such as, for example, a compound TNO155 as a SHP2 inhibitor and a PD-1 inhibitor, producing an effect, for example, slowing the symptomatic progression of a proliferative disease, particularly cancer, or symptoms thereof, which is greater than the simple addition of the effects of each drug administered by themselves. A synergistic effect can be calculated, for example, using suitable methods such as the Sigmoid-Emax equation (Holford, N. H. G. and Scheiner, L. B., Clin. Pharmacokinet. 6: 429-453 (1981)), the equation of Loewe additivity (Loewe, S. and Muischnek, H., Arch. Exp. Pathol Pharmacol. 114: 313-326 (1926)) and the median-effect equation (Chou, T. C. and Talalay, P., Adv. Enzyme Regul. 22: 27-55 (1984)). Each equation referred to above can be applied to experimental data to generate a corresponding graph to aid in assessing the effects of the drug combination. The corresponding graphs associated with the equations referred to above are the concentration-effect curve, isobologram curve and combination index curve, respectively.

The term "pharmaceutical combination" as used herein refers to either a fixed combination in one dosage unit form, or non-fixed combination or a kit of parts for the combined administration where two or more therapeutic agents may be administered independently at the same time or separately within time intervals, especially where these time intervals allow that the combination partners show a cooperative, *e.g.* synergistic effect.

PD-1 inhibitors include PDR001. PDR001 is also known as spartalizumab, an anti-PD-1 antibody molecule described in US 2015/0210769, published on July 30, 2015, entitled "Antibody Molecules to PD-1 and Uses Thereof".

Further anti-PD-1 antibody molecules include the following:
Nivolumab (Bristol-Myers Squibb), also known as MDX-1106, MDX-1106-04, ONO-4538, BMS-936558, or OPDIVO^{®}. Nivolumab (clone 5C4) and other anti-PD-1 antibodies are disclosed in US 8,008,449 and WO 2006/121168;
Pembrolizumab (Merck & Co), also known as Lambrolizumab, MK-3475, MK03475, SCH-900475, or KEYTRUDA^{®}. Pembrolizumab and other anti-PD-1 antibodies are disclosed in Hamid, O. et al. (2013) New England Journal of Medicine 369 (2): 134-44, US 8,354,509, and WO 2009/114335;
Pidilizumab (CureTech), also known as CT-011. Pidilizumab and other anti-PD-1 antibodies are disclosed in Rosenblatt, J. et al. (2011) J Immunotherapy 34(5): 409-18, US 7,695,715, US 7,332,582, and US 8,686,119;
MEDI0680 (Medimmune), also known as AMP-514. MEDI0680 and other anti-PD-1 antibodies are disclosed in US 9,205,148 and WO 2012/145493 entirety;
AMP-224 (B7-DCIg (Amplimmune), e.g., disclosed in WO 2010/027827 and WO 2011/066342;
REGN2810 (Regeneron); PF-06801591 (Pfizer); BGB-A317 or BGB-108 (Beigene); INCSHR1210 (Incyte), also known as INCSHR01210 or SHR-1210; TSR-042 (Tesaro), also known as ANB011; and further known anti-PD-1 antibodies including those described, *e.g.,* in WO 2015/112800, WO 2016/092419, WO 2015/085847, WO 2014/179664, WO 2014/194302, WO 2014/209804, WO 2015/200119, US 8,735,553, US 7,488,802, US 8,927,697, US 8,993,731, and US 9,102,727.

A combination of the invention, TNO155 and a PD-1 inhibitor, is also intended to represent unlabeled forms as well as isotopically labeled forms of the compounds. Isotopically labeled compounds have one or more atoms replaced by an atom having a selected atomic mass or mass number. Examples of isotopes that can be incorporated into TNO155 and a PD-1 inhibitor include isotopes, where possible, of hydrogen, carbon, nitrogen, oxygen, and chlorine, for example, ²H, ³H, ¹¹C, ¹³C, ¹⁴C, ¹⁵N, ³⁵S, ³⁶Cl. The invention includes isotopically labeled TNO155 and a PD-1 inhibitor, for example into which radioactive isotopes, such as ³H and ¹⁴C, or nonradioactive isotopes, such as ²H and ¹³C, are present. Isotopically labelled TNO155 and a PD-1 inhibitor are useful in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. Isotopically-labeled compounds of the invention can generally be prepared by conventional techniques known to those skilled in the art or by processes analogous to those described in the accompanying Examples using appropriate isotopically-labeled reagents.

Further, substitution with heavier isotopes, particularly deuterium (i.e., ²H or D) may afford certain therapeutic advantages resulting from greater metabolic stability, for example increased in vivo half-life or reduced dosage requirements or an improvement in therapeutic index. It is understood that deuterium in this context is regarded as a substituent of either TNO155 or a PD-1 inhibitor. The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in TNO155 or a PD-1 inhibitor is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

### Description of Preferred Embodiments

TNO155 is an orally bioavailable small molecule inhibitor of SHP2 activity. SHP2 transduces signaling downstream of activated RTKs, as well as of PD-1 and other immunoreceptors, for example, CTLA4, SCF1R and LILRB4. In preclinical models, tumor dependence on RTKs predicts dependence on SHP2. Further, based on preclinical data, SHP2 inhibition can enhance the anti-tumor activity of immune checkpoint inhibitors.

In one embodiment, with respect to the pharmaceutical combination of the invention, is a pharmaceutical combination comprising (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and a PD-1 inhibitor, or a pharmaceutically acceptable salt thereof.

In a further embodiment, (3S,4S)-8-(6-amino-5-((2-amino-3-chloropyridin-4-yl)thio)pyrazin-2-yl)-3-methyl-2-oxa-8-azaspiro[4.5]decan-4-amine, or a pharmaceutically acceptable salt thereof, and a PD-1 inhibitor, or a pharmaceutically acceptable salt thereof, are administered separately, simultaneously or sequentially, in any order.

In a further embodiment, (3S,4S)-8-(6-amino-5-((2-amino-3-chloropyridin-4-yl)thio)pyrazin-2-yl)-3-methyl-2-oxa-8-azaspiro[4.5]decan-4-amine is in an oral dose form.

In another embodiment, is a pharmaceutical composition comprising a pharmaceutical combination of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and a PD-1 inhibitor and at least one pharmaceutically acceptable carrier.

In another embodiment, the PD-1 inhibitor is an anti-PD-1 antibody molecule.

In a further embodiment, the PD-1 inhibitor is an anti-PD-1 antibody molecule as described in US 2015/0210769, published on July 30, 2015, entitled "Antibody Molecules to PD-1 and Uses Thereof". In some embodiments, the anti-PD-1 antibody molecule is BAP049-Clone E or BAP049-Clone B.

In a further embodiment, the anti-PD-1 antibody molecule is Spartalizumab (PDR001).

In one embodiment, the anti-PD-1 antibody molecule comprises at least one, two, three, four, five or six complementarity determining regions (CDRs) (or collectively all of the CDRs) from a heavy and light chain variable region comprising an amino acid sequence shown in Table 1 (*e.g*., from the heavy and light chain variable region sequences of BAP049-Clone-E or BAP049-Clone-B disclosed in Table 1), or encoded by a nucleotide sequence shown in Table 1. In some embodiments, the CDRs are according to the Kabat definition (*e.g*., as set out in Table 1). In some embodiments, the CDRs are according to the Chothia definition (*e.g*., as set out in Table 1). In some embodiments, the CDRs are according to the combined CDR definitions of both Kabat and Chothia (*e.g*., as set out in Table 1). In one embodiment, the combination of Kabat and Chothia CDR of VH CDR1 comprises the amino acid sequence GYTFTTYWMH (SEQ ID NO: 541). In one embodiment, one or more of the CDRs (or collectively all of the CDRs) have one, two, three, four, five, six or more changes, *e.g.,* amino acid substitutions (*e.g.,* conservative amino acid substitutions) or deletions, relative to an amino acid sequence shown in Table 1, or encoded by a nucleotide sequence shown in Table 1.

In one embodiment, the anti-PD-1 antibody molecule comprises a heavy chain variable region (VH) comprising a VHCDR1 amino acid sequence of SEQ ID NO: 501, a VHCDR2 amino acid sequence of SEQ ID NO: 502, and a VHCDR3 amino acid sequence of SEQ ID NO: 503; and a light chain variable region (VL) comprising a VLCDR1 amino acid sequence of SEQ ID NO: 510, a VLCDR2 amino acid sequence of SEQ ID NO: 511, and a VLCDR3 amino acid sequence of SEQ ID NO: 512, each disclosed in Table 1.

In one embodiment, the antibody molecule comprises a VH comprising a VHCDR1 encoded by the nucleotide sequence of SEQ ID NO: 524, a VHCDR2 encoded by the nucleotide sequence of SEQ ID NO: 525, and a VHCDR3 encoded by the nucleotide sequence of SEQ ID NO: 526; and a VL comprising a VLCDR1 encoded by the nucleotide sequence of SEQ ID NO: 529, a VLCDR2 encoded by the nucleotide sequence of SEQ ID NO: 530, and a VLCDR3 encoded by the nucleotide sequence of SEQ ID NO: 531, each disclosed in Table 1.

In one embodiment, the anti-PD-1 antibody molecule comprises a VH comprising the amino acid sequence of SEQ ID NO: 506, or an amino acid sequence at least 85%, 90%, 95%, or 99% identical or higher to SEQ ID NO: 506. In one embodiment, the anti-PD-1 antibody molecule comprises a VL comprising the amino acid sequence of SEQ ID NO: 520, or an amino acid sequence at least 85%, 90%, 95%, or 99% identical or higher to SEQ ID NO: 520. In one embodiment, the anti-PD-1 antibody molecule comprises a VL comprising the amino acid sequence of SEQ ID NO: 516, or an amino acid sequence at least 85%, 90%, 95%, or 99% identical or higher to SEQ ID NO: 516. In one embodiment, the anti-PD-1 antibody molecule comprises a VH comprising the amino acid sequence of SEQ ID NO: 506 and a VL comprising the amino acid sequence of SEQ ID NO: 520. In one embodiment, the anti-PD-1 antibody molecule comprises a VH comprising the amino acid sequence of SEQ ID NO: 506 and a VL comprising the amino acid sequence of SEQ ID NO: 516.

In one embodiment, the antibody molecule comprises a VH encoded by the nucleotide sequence of SEQ ID NO: 507, or a nucleotide sequence at least 85%, 90%, 95%, or 99% identical or higher to SEQ ID NO: 507. In one embodiment, the antibody molecule comprises a VL encoded by the nucleotide sequence of SEQ ID NO: 521 or 517, or a nucleotide sequence at least 85%, 90%, 95%, or 99% identical or higher to SEQ ID NO: 521 or 517. In one embodiment, the antibody molecule comprises a VH encoded by the nucleotide sequence of SEQ ID NO: 507 and a VL encoded by the nucleotide sequence of SEQ ID NO: 521 or 517.

In one embodiment, the anti-PD-1 antibody molecule comprises a heavy chain comprising the amino acid sequence of SEQ ID NO: 508, or an amino acid sequence at least 85%, 90%, 95%, or 99% identical or higher to SEQ ID NO: 508. In one embodiment, the anti-PD-1 antibody molecule comprises a light chain comprising the amino acid sequence of SEQ ID NO: 522, or an amino acid sequence at least 85%, 90%, 95%, or 99% identical or higher to SEQ ID NO: 522. In one embodiment, the anti-PD-1 antibody molecule comprises a light chain comprising the amino acid sequence of SEQ ID NO: 518, or an amino acid sequence at least 85%, 90%, 95%, or 99% identical or higher to SEQ ID NO: 518. In one embodiment, the anti-PD-1 antibody molecule comprises a heavy chain comprising the amino acid sequence of SEQ ID NO: 508 and a light chain comprising the amino acid sequence of SEQ ID NO: 522. In one embodiment, the anti-PD-1 antibody molecule comprises a heavy chain comprising the amino acid sequence of SEQ ID NO: 508 and a light chain comprising the amino acid sequence of SEQ ID NO: 518.

In one embodiment, the antibody molecule comprises a heavy chain encoded by the nucleotide sequence of SEQ ID NO: 509, or a nucleotide sequence at least 85%, 90%, 95%, or 99% identical or higher to SEQ ID NO: 509. In one embodiment, the antibody molecule comprises a light chain encoded by the nucleotide sequence of SEQ ID NO: 523 or 519, or a nucleotide sequence at least 85%, 90%, 95%, or 99% identical or higher to SEQ ID NO: 523 or 519. In one embodiment, the antibody molecule comprises a heavy chain encoded by the nucleotide sequence of SEQ ID NO: 509 and a light chain encoded by the nucleotide sequence of SEQ ID NO: 523 or 519.

The antibody molecules described herein can be made by vectors, host cells, and methods described in US 2015/0210769.

**Table 1. Amino acid and nucleotide sequences of exemplary anti-PD-1 antibody molecules**

| **BAP049-Clone-B HC** | | |
|---|---|---|
| SEQ ID NO: 501 (Kabat) | HCDR1 | TYWMH |
| SEQ ID NO: 502 (Kabat) | HCDR2 | NIYPGTGGSNFDEKFKN |
| SEQ ID NO: 503 (Kabat) | HCDR3 | WTTGTGAY |
| SEQ ID NO: 504 (Chothia) | HCDR1 | GYTFTTY |
| SEQ ID NO: 505 (Chothia) | HCDR2 | YPGTGG |
| SEQ ID NO: 503 (Chothia) | HCDR3 | WTTGTGAY |
| SEQ ID NO: 506 | VH | EVQLVQSGAEVKKPGESLRISCKGSGYTFTTYWMHWVRQ ATGQGLEWMGNIYPGTGGSNFDEKFKNRVTITADKSTSTA YMELSSLRSEDTAVYYCTRWTTGTGAYWGQGTTVTVSS |
| SEQ ID NO: 507 | DNA VH | GAGGTGCAGCTGGTGCAGTCAGGCGCCGAAGTGAAGAA GCCCGGCGAGTCACTGAGAATTAGCTGTAAAGGTTCAG GCTACACCTTCACTACCTACTGGATGCACTGGGTCCGCC AGGCTACCGGTCAAGGCCTCGAGTGGATGGGTAATATC TACCCCGGCACCGGCGGCTCTAACTTCGACGAGAAGTTT AAGAATAGAGTGACTATCACCGCCGATAAGTCTACTAG CACCGCCTATATGGAACTGTCTAGCCTGAGATCAGAGG ACACCGCCGTCTACTACTGCACTAGGTGGACTACCGGCA CAGGCGCCTACTGGGGTCAAGGCACTACCGTGACCGTG TCTAGC |
| SEQ ID NO: 508 | Heavy chain | EVQLVQSGAEVKKPGESLRISCKGSGYTFTTYWMHWVRQ ATGQGLEWMGNIYPGTGGSNFDEKFKNRVTITADKSTSTA YMELSSLRSEDTAVYYCTRWTTGTGAYWGQGTTVTVSSA STKGPSVFPLAPCSRSTSESTAALGCLVKDYFPEPVTVSWN SGALTSGVHTFPAVLQSSGLYSLSSVVTVPSSSLGTKTYTC NVDHKPSNTKVDKRVESKYGPPCPPCPAPEFLGGPSVFLFP PKPKDTLMISRTPEVTCVVVDVSQEDPEVQFNWYVDGVE |
| | | VHNAKTKPREEQFNSTYRVVSVLTVLHQDWLNGKEYKCK VSNKGLPSSIEKTISKAKGQPREPQVYTLPPSQEEMTKNQV SLTCLVKGFYPSDIAVEWESNGQPENNYKTTPPVLDSDGSF FLYSRLTVDKSRWQEGNVFSCSVMHEALHNHYTQKSLSLS LG |
| SEQ ID NO: 509 | DNA heavy chain | GAGGTGCAGCTGGTGCAGTCAGGCGCCGAAGTGAAGAA GCCCGGCGAGTCACTGAGAATTAGCTGTAAAGGTTCAG GCTACACCTTCACTACCTACTGGATGCACTGGGTCCGCC AGGCTACCGGTCAAGGCCTCGAGTGGATGGGTAATATC TACCCCGGCACCGGCGGCTCTAACTTCGACGAGAAGTTT AAGAATAGAGTGACTATCACCGCCGATAAGTCTACTAG CACCGCCTATATGGAACTGTCTAGCCTGAGATCAGAGG ACACCGCCGTCTACTACTGCACTAGGTGGACTACCGGCA CAGGCGCCTACTGGGGTCAAGGCACTACCGTGACCGTG TCTAGCGCTAGCACTAAGGGCCCGTCCGTGTTCCCCCTG GCACCTTGTAGCCGGAGCACTAGCGAATCCACCGCTGCC CTCGGCTGCCTGGTCAAGGATTACTTCCCGGAGCCCGTG ACCGTGTCCTGGAACAGCGGAGCCCTGACCTCCGGAGT GCACACCTTCCCCGCTGTGCTGCAGAGCTCCGGGCTGTA CTCGCTGTCGTCGGTGGTCACGGTGCCTTCATCTAGCCT GGGTACCAAGACCTACACTTGCAACGTGGACCACAAGC CTTCCAACACTAAGGTGGACAAGCGCGTCGAATCGAAG TACGGCCCACCGTGCCCGCCTTGTCCCGCGCCGGAGTTC CTCGGCGGTCCCTCGGTCTTTCTGTTCCCACCGAAGCCC AAGGACACTTTGATGATTTCCCGCACCCCTGAAGTGACA TGCGTGGTCGTGGACGTGTCACAGGAAGATCCGGAGGT GCAGTTCAATTGGTACGTGGATGGCGTCGAGGTGCACA ACGCCAAAACCAAGCCGAGGGAGGAGCAGTTCAACTCC ACTTACCGCGTCGTGTCCGTGCTGACGGTGCTGCATCAG GACTGGCTGAACGGGAAGGAGTACAAGTGCAAAGTGTC CAACAAGGGACTTCCTAGCTCAATCGAAAAGACCATCT CGAAAGCCAAGGGACAGCCCCGGGAACCCCAAGTGTAT ACCCTGCCACCGAGCCAGGAAGAAATGACTAAGAACCA AGTCTCATTGACTTGCCTTGTGAAGGGCTTCTACCCATC GGATATCGCCGTGGAATGGGAGTCCAACGGCCAGCCGG AAAACAACTACAAGACCACCCCTCCGGTGCTGGACTCA GACGGATCCTTCTTCCTCTACTCGCGGCTGACCGTGGAT AAGAGCAGATGGCAGGAGGGAAATGTGTTCAGCTGTTC |
| | | TGTGATGCATGAAGCCCTGCACAACCACTACACTCAGA AGTCCCTGTCCCTCTCCCTGGGA |

| **BAP049-Clone-B LC** | | |
|---|---|---|
| SEQ ID NO: 510 (Kabat) | LCDR1 | KSSQSLLDSGNQKNFLT |
| SEQ ID NO: 511 (Kabat) | LCDR2 | WASTRES |
| SEQ ID NO: 512 (Kabat) | LCDR3 | QNDYSYPYT |
| SEQ ID NO: 513 (Chothia) | LCDR1 | SQSLLDSGNQKNF |
| SEQ ID NO: 514 (Chothia) | LCDR2 | WAS |
| SEQ ID NO: 515 (Chothia) | LCDR3 | DYSYPY |
| SEQ ID NO: 516 | VL | EIVLTQSPATLSLSPGERATLSCKSSQSLLDSGNQKNFLTW YQQKPGKAPKLLIYWASTRESGVPSRFSGSGSGTDFTFTISS LQPEDIATYYCQNDYSYPYTFGQGTKVEIK |
| SEQ ID NO: 517 | DNA VL | GAGATCGTCCTGACTCAGTCACCCGCTACCCTGAGCCTG AGCCCTGGCGAGCGGGCTACACTGAGCTGTAAATCTAG TCAGTCACTGCTGGATAGCGGTAATCAGAAGAACTTCCT GACCTGGTATCAGCAGAAGCCCGGTAAAGCCCCTAAGC TGCTGATCTACTGGGCCTCTACTAGAGAATCAGGCGTGC CCTCTAGGTTTAGCGGTAGCGGTAGTGGCACCGACTTCA CCTTCACTATCTCTAGCCTGCAGCCCGAGGATATCGCTA CCTACTACTGTCAGAACGACTATAGCTACCCCTACACCT TCGGTCAAGGCACTAAGGTCGAGATTAAG |
| SEQ ID NO: 518 | Light chain | EIVLTQSPATLSLSPGERATLSCKSSQSLLDSGNQKNFLTW YQQKPGKAPKLLIYWASTRESGVPSRFSGSGSGTDFTFTISS LQPEDIATYYCQNDYSYPYTFGQGTKVEIKRTVAAPSVFIF PPSDEQLKSGTASVVCLLNNFYPREAKVQWKVDNALQSG NSQESVTEQDSKDSTYSLSSTLTLSKADYEKHKVYACEVT HQGLSSPVTKSFNRGEC |
| SEQ ID NO: 519 | DNA light chain | GAGATCGTCCTGACTCAGTCACCCGCTACCCTGAGCCTG AGCCCTGGCGAGCGGGCTACACTGAGCTGTAAATCTAG TCAGTCACTGCTGGATAGCGGTAATCAGAAGAACTTCCT GACCTGGTATCAGCAGAAGCCCGGTAAAGCCCCTAAGC TGCTGATCTACTGGGCCTCTACTAGAGAATCAGGCGTGC CCTCTAGGTTTAGCGGTAGCGGTAGTGGCACCGACTTCA CCTTCACTATCTCTAGCCTGCAGCCCGAGGATATCGCTA CCTACTACTGTCAGAACGACTATAGCTACCCCTACACCT |
| | | TCGGTCAAGGCACTAAGGTCGAGATTAAGCGTACGGTG GCCGCTCCCAGCGTGTTCATCTTCCCCCCCAGCGACGAG CAGCTGAAGAGCGGCACCGCCAGCGTGGTGTGCCTGCT GAACAACTTCTACCCCCGGGAGGCCAAGGTGCAGTGGA AGGTGGACAACGCCCTGCAGAGCGGCAACAGCCAGGAG AGCGTCACCGAGCAGGACAGCAAGGACTCCACCTACAG CCTGAGCAGCACCCTGACCCTGAGCAAGGCCGACTACG AGAAGCATAAGGTGTACGCCTGCGAGGTGACCCACCAG GGCCTGTCCAGCCCCGTGACCAAGAGCTTCAACAGGGG CGAGTGC |

| **BAP049-Clone-E HC** | | |
|---|---|---|
| SEQ ID NO: 501 (Kabat) | HCDR1 | TYWMH |
| SEQ ID NO: 502 (Kabat) | HCDR2 | NIYPGTGGSNFDEKFKN |
| SEQ ID NO: 503 (Kabat) | HCDR3 | WTTGTGAY |
| SEQ ID NO: 504 (Chothia) | HCDR1 | GYTFTTY |
| SEQ ID NO: 505 (Chothia) | HCDR2 | YPGTGG |
| SEQ ID NO: 503 (Chothia) | HCDR3 | WTTGTGAY |
| SEQ ID NO: 506 | VH | EVQLVQSGAEVKKPGESLRISCKGSGYTFTTYWMHWVRQ ATGQGLEWMGNIYPGTGGSNFDEKFKNRVTITADKSTSTA YMELSSLRSEDTAVYYCTRWTTGTGAYWGQGTTVTVSS |
| SEQ ID NO: 507 | DNA VH | GAGGTGCAGCTGGTGCAGTCAGGCGCCGAAGTGAAGAA GCCCGGCGAGTCACTGAGAATTAGCTGTAAAGGTTCAG GCTACACCTTCACTACCTACTGGATGCACTGGGTCCGCC AGGCTACCGGTCAAGGCCTCGAGTGGATGGGTAATATC TACCCCGGCACCGGCGGCTCTAACTTCGACGAGAAGTTT AAGAATAGAGTGACTATCACCGCCGATAAGTCTACTAG CACCGCCTATATGGAACTGTCTAGCCTGAGATCAGAGG ACACCGCCGTCTACTACTGCACTAGGTGGACTACCGGCA CAGGCGCCTACTGGGGTCAAGGCACTACCGTGACCGTG TCTAGC |
| SEQ ID NO: 508 | Heavy chain | EVQLVQSGAEVKKPGESLRISCKGSGYTFTTYWMHWVRQ ATGQGLEWMGNIYPGTGGSNFDEKFKNRVTITADKSTSTA YMELSSLRSEDTAVYYCTRWTTGTGAYWGQGTTVTVSSA STKGPSVFPLAPCSRSTSESTAALGCLVKDYFPEPVTVSWN SGALTSGVHTFPAVLQSSGLYSLSSVVTVPSSSLGTKTYTC |
| | | NVDHKPSNTKVDKRVESKYGPPCPPCPAPEFLGGPSVFLFP PKPKDTLMISRTPEVTCVVVDVSQEDPEVQFNWYVDGVE VHNAKTKPREEQFNSTYRVVSVLTVLHQDWLNGKEYKCK VSNKGLPSSIEKTISKAKGQPREPQVYTLPPSQEEMTKNQV SLTCLVKGFYPSDIAVEWESNGQPENNYKTTPPVLDSDGSF FLYSRLTVDKSRWQEGNVFSCSVMHEALHNHYTQKSLSLS LG |
| SEQ ID NO: 509 | DNA heavy chain | GAGGTGCAGCTGGTGCAGTCAGGCGCCGAAGTGAAGAA GCCCGGCGAGTCACTGAGAATTAGCTGTAAAGGTTCAG GCTACACCTTCACTACCTACTGGATGCACTGGGTCCGCC AGGCTACCGGTCAAGGCCTCGAGTGGATGGGTAATATC TACCCCGGCACCGGCGGCTCTAACTTCGACGAGAAGTTT AAGAATAGAGTGACTATCACCGCCGATAAGTCTACTAG CACCGCCTATATGGAACTGTCTAGCCTGAGATCAGAGG ACACCGCCGTCTACTACTGCACTAGGTGGACTACCGGCA CAGGCGCCTACTGGGGTCAAGGCACTACCGTGACCGTG TCTAGCGCTAGCACTAAGGGCCCGTCCGTGTTCCCCCTG GCACCTTGTAGCCGGAGCACTAGCGAATCCACCGCTGCC CTCGGCTGCCTGGTCAAGGATTACTTCCCGGAGCCCGTG ACCGTGTCCTGGAACAGCGGAGCCCTGACCTCCGGAGT GCACACCTTCCCCGCTGTGCTGCAGAGCTCCGGGCTGTA CTCGCTGTCGTCGGTGGTCACGGTGCCTTCATCTAGCCT GGGTACCAAGACCTACACTTGCAACGTGGACCACAAGC CTTCCAACACTAAGGTGGACAAGCGCGTCGAATCGAAG TACGGCCCACCGTGCCCGCCTTGTCCCGCGCCGGAGTTC CTCGGCGGTCCCTCGGTCTTTCTGTTCCCACCGAAGCCC AAGGACACTTTGATGATTTCCCGCACCCCTGAAGTGACA TGCGTGGTCGTGGACGTGTCACAGGAAGATCCGGAGGT GCAGTTCAATTGGTACGTGGATGGCGTCGAGGTGCACA ACGCCAAAACCAAGCCGAGGGAGGAGCAGTTCAACTCC ACTTACCGCGTCGTGTCCGTGCTGACGGTGCTGCATCAG GACTGGCTGAACGGGAAGGAGTACAAGTGCAAAGTGTC CAACAAGGGACTTCCTAGCTCAATCGAAAAGACCATCT CGAAAGCCAAGGGACAGCCCCGGGAACCCCAAGTGTAT ACCCTGCCACCGAGCCAGGAAGAAATGACTAAGAACCA AGTCTCATTGACTTGCCTTGTGAAGGGCTTCTACCCATC GGATATCGCCGTGGAATGGGAGTCCAACGGCCAGCCGG AAAACAACTACAAGACCACCCCTCCGGTGCTGGACTCA |
| | | GACGGATCCTTCTTCCTCTACTCGCGGCTGACCGTGGAT AAGAGCAGATGGCAGGAGGGAAATGTGTTCAGCTGTTC TGTGATGCATGAAGCCCTGCACAACCACTACACTCAGA AGTCCCTGTCCCTCTCCCTGGGA |

| **BAP049-Clone-E LC** | | |
|---|---|---|
| SEQ ID NO: 510 (Kabat) | LCDR1 | KSSQSLLDSGNQKNFLT |
| SEQ ID NO: 511 (Kabat) | LCDR2 | WASTRES |
| SEQ ID NO: 512 (Kabat) | LCDR3 | QNDYSYPYT |
| SEQ ID NO: 513 (Chothia) | LCDR1 | SQSLLDSGNQKNF |
| SEQ ID NO: 514 (Chothia) | LCDR2 | WAS |
| SEQ ID NO: 515 (Chothia) | LCDR3 | DYSYPY |
| SEQ ID NO: 520 | VL | EIVLTQSPATLSLSPGERATLSCKSSQSLLDSGNQKNFLTW YQQKPGQAPRLLIYWASTRESGVPSRFSGSGSGTDFTFTISS LEAEDAATYYCQNDYSYPYTFGQGTKVEIK |
| SEQ ID NO: 521 | DNA VL | GAGATCGTCCTGACTCAGTCACCCGCTACCCTGAGCCTG AGCCCTGGCGAGCGGGCTACACTGAGCTGTAAATCTAG TCAGTCACTGCTGGATAGCGGTAATCAGAAGAACTTCCT GACCTGGTATCAGCAGAAGCCCGGTCAAGCCCCTAGAC TGCTGATCTACTGGGCCTCTACTAGAGAATCAGGCGTGC CCTCTAGGTTTAGCGGTAGCGGTAGTGGCACCGACTTCA CCTTCACTATCTCTAGCCTGGAAGCCGAGGACGCCGCTA CCTACTACTGTCAGAACGACTATAGCTACCCCTACACCT TCGGTCAAGGCACTAAGGTCGAGATTAAG |
| SEQ ID NO: 522 | Light chain | EIVLTQSPATLSLSPGERATLSCKSSQSLLDSGNQKNFLTW YQQKPGQAPRLLIYWASTRESGVPSRFSGSGSGTDFTFTISS LEAEDAATYYCQNDYSYPYTFGQGTKVEIKRTVAAPSVFIF PPSDEQLKSGTASVVCLLNNFYPREAKVQWKVDNALQSG NSQESVTEQDSKDSTYSLSSTLTLSKADYEKHKVYACEVT HQGLSSPVTKSFNRGEC |
| SEQ ID NO: 523 | DNA light chain | GAGATCGTCCTGACTCAGTCACCCGCTACCCTGAGCCTG AGCCCTGGCGAGCGGGCTACACTGAGCTGTAAATCTAG TCAGTCACTGCTGGATAGCGGTAATCAGAAGAACTTCCT GACCTGGTATCAGCAGAAGCCCGGTCAAGCCCCTAGAC TGCTGATCTACTGGGCCTCTACTAGAGAATCAGGCGTGC CCTCTAGGTTTAGCGGTAGCGGTAGTGGCACCGACTTCA |
| | | CCTTCACTATCTCTAGCCTGGAAGCCGAGGACGCCGCTA CCTACTACTGTCAGAACGACTATAGCTACCCCTACACCT TCGGTCAAGGCACTAAGGTCGAGATTAAGCGTACGGTG GCCGCTCCCAGCGTGTTCATCTTCCCCCCCAGCGACGAG CAGCTGAAGAGCGGCACCGCCAGCGTGGTGTGCCTGCT GAACAACTTCTACCCCCGGGAGGCCAAGGTGCAGTGGA AGGTGGACAACGCCCTGCAGAGCGGCAACAGCCAGGAG AGCGTCACCGAGCAGGACAGCAAGGACTCCACCTACAG CCTGAGCAGCACCCTGACCCTGAGCAAGGCCGACTACG AGAAGCATAAGGTGTACGCCTGCGAGGTGACCCACCAG GGCCTGTCCAGCCCCGTGACCAAGAGCTTCAACAGGGG CGAGTGC |

| **BAP049-Clone-B HC** | | |
|---|---|---|
| SEQ ID NO: 524 (Kabat) | HCDR1 | ACCTACTGGATGCAC |
| SEQ ID NO: 525 (Kabat) | HCDR2 | AATATCTACCCCGGCACCGGCGGCTCTAACTTCGACGAG AAGTTTAAGAAT |
| SEQ ID NO: 526 (Kabat) | HCDR3 | TGGACTACCGGCACAGGCGCCTAC |
| SEQ ID NO: 527 (Chothia) | HCDR1 | GGCTACACCTTCACTACCTAC |
| SEQ ID NO: 528 (Chothia) | HCDR2 | TACCCCGGCACCGGCGGC |
| SEQ ID NO: 526 (Chothia) | HCDR3 | TGGACTACCGGCACAGGCGCCTAC |

| **BAP049-Clone-B LC** | | |
|---|---|---|
| SEQ ID NO: 529 (Kabat) | LCDR1 | AAATCTAGTCAGTCACTGCTGGATAGCGGTAATCAGAA GAACTTCCTGACC |
| SEQ ID NO: 530 (Kabat) | LCDR2 | TGGGCCTCTACTAGAGAATCA |
| SEQ ID NO: 531 (Kabat) | LCDR3 | CAGAACGACTATAGCTACCCCTACACC |
| SEQ ID NO: 532 (Chothia) | LCDR1 | AGTCAGTCACTGCTGGATAGCGGTAATCAGAAGAACTT C |
| SEQ ID NO: 533 (Chothia) | LCDR2 | TGGGCCTCT |
| SEQ ID NO: 534 (Chothia) | LCDR3 | GACTATAGCTACCCCTAC |
| **BAP049-Clone-E HC** | | |
| SEQ ID NO: 524 (Kabat) | HCDR1 | ACCTACTGGATGCAC |
| SEQ ID NO: 525 (Kabat) | HCDR2 | AATATCTACCCCGGCACCGGCGGCTCTAACTTCGACGAG AAGTTTAAGAAT |
| SEQ ID NO: 526 (Kabat) | HCDR3 | TGGACTACCGGCACAGGCGCCTAC |
| SEQ ID NO: 527 (Chothia) | HCDR1 | GGCTACACCTTCACTACCTAC |
| SEQ ID NO: 528 (Chothia) | HCDR2 | TACCCCGGCACCGGCGGC |
| SEQ ID NO: 526 (Chothia) | HCDR3 | TGGACTACCGGCACAGGCGCCTAC |

| **BAP049-Clone-E LC** | | |
|---|---|---|
| SEQ ID NO: 529 (Kabat) | LCDR1 | AAATCTAGTCAGTCACTGCTGGATAGCGGTAATCAGAA GAACTTCCTGACC |
| SEQ ID NO: 530 (Kabat) | LCDR2 | TGGGCCTCTACTAGAGAATCA |
| SEQ ID NO: 531 (Kabat) | LCDR3 | CAGAACGACTATAGCTACCCCTACACC |
| SEQ ID NO: 532 (Chothia) | LCDR1 | AGTCAGTCACTGCTGGATAGCGGTAATCAGAAGAACTT C |
| SEQ ID NO: 533 (Chothia) | LCDR2 | TGGGCCTCT |
| SEQ ID NO: 534 (Chothia) | LCDR3 | GACTATAGCTACCCCTAC |

In a further embodiment, the cancer is selected from esophageal squamous cell carcinoma, head and neck squamous cell carcinoma, colorectal cancer, ovarian cancer, pancreatic cancer, non-small cell lung cancer and renal cell carcinoma.

In a further embodiment, the cancer is selected from esophageal squamous cell carcinoma and pharyngeal squamous cell carcinoma.

In a further embodiment, the cancer is colorectal cancer.

In a further embodiment, the cancer is non-small cell lung cancer.

In a further embodiment, the cancer is head and neck squamous cell carcinoma.

In a further embodiment, (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and the second therapeutic agent are administered simultaneously, separately or over a period of time.

In a further embodiment, the amount of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, administered to the subject in need therof is effective to treat the cancer.

In a further embodiment, the amounts of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and the second therapeutic agent, administered to the subject in need thereof is effective to treat the cancer.

In a further embodiment, the second therapeutic agent is an immunomodulator.

In a further embodiment, the second therapeutic agent is an immune checkpoint inhibitor.

In a further embodiment, the second therapeutic agent is a PD-1 inhibitor.

In a further embodiment, the PD-1 inhibitor is selected from PDR001, Nivolumab, Pembrolizumab, Pidilizumab, MEDI0680, REGN2810, TSR-042, PF-06801591, BGB-A317, BGB-108, INCSHR1210, or AMP-224.

In a further embodiment, the PD-1 inhibitor is PDR001.

In a further embodiment, (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, is administered orally at a dose of about 1.5 mg per day, or 3 mg per day, or 6 mg per day, or 10 mg per day, or 20 mg per day, or 30 mg per day, or 40 mg per day, or 50 mg per day, or 60 mg per day, or 70 mg per day, or 80 mg per day, or 90 mg per day, or 100 mg per day.

In a further embodiment, the dose per day of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, is on a 21 day cycle of 2 weeks on drug followed by 1 week off drug.

In a further embodiment, the dose is 20 mg QD per day of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, is on a 21 day cycle of 2 weeks on drug followed by 1 week off drug.

In a further embodiment, PDR001 is administered at a dose of about 300 mg once every 3 weeks.

In a further embodiment, PDR001 is administered at a dose of about 400 mg once every 4 weeks.

In a further embodiment, the dose per day of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, is on a 21 day cycle of 2 weeks on drug followed by 1 week off drug.

In a further embodiment, the dose is 20 mg QD per day of (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, is on a 21 day cycle of 2 weeks on drug followed by 1 week off drug.

In a further embodiment, the cancer is selected from esophageal squamous cell carcinoma, head and neck squamous cell carcinoma, colorectal cancer, ovarian cancer, pancreatic cancer, non-small cell lung cancer and renal cell carcinoma.

In a further embodiment, the cancer is colorectal cancer.

In a further embodiment, the cancer is non-small cell lung cancer.

In a further embodiment, the cancer is head and neck squamous cell carcinoma.

In a further embodiment, (3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and the second therapeutic agent are administered simultaneously, separately, or over a period of time.

In a further embodiment, the second therapeutic agent is an immunomodulator.

In a further embodiment, the second therapeutic agent is an immune checkpoint inhibitor.

In a further embodiment, the second therapeutic agent is a PD-1 inhibitor.

In a further embodiment, the PD-1 inhibitor is selected from PDR001, Nivolumab, Pembrolizumab, Pidilizumab, MEDI0680, REGN2810, TSR-042, PF-06801591, BGB-A317, BGB-108, INCSHR1210, or AMP-224.

In a further embodiment, the PD-1 inhibitor is PDR001.

In a further embodiment, PDR001 is administered at a dose of about 300 mg once every 3 weeks.

In a further embodiment, PDR001 is administered at a dose of about 400 mg once every 4 weeks.

In a further embodiment, the second therapeutic agent is administered intravenously.

In another embodiment, is a pharmaceutical combination of (3 S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and PDR001, for use in the treatment of: esophageal or head and neck squamous cell carcinoma; colorectal, ovarian, pancreatic or non-small cell lung cancer; and renal cell carcinoma.

In a further embodiment, the cancer is colorectal cancer.

In a further embodiment, the cancer is non-small cell lung cancer.

In a further embodiment, the cancer is head and neck squamous cell carcinoma.

In another embodiment, is a use of the pharmaceutical combination of ((3S,4S)-8-(6-amino-5-((2-amino-3-chloropyridin-4-yl)thio)pyrazin-2-yl)-3-methyl-2-oxa-8-azaspiro[4.5]decan-4-amine, or pharmaceutically acceptable salt thereof, and PDR001, for the manufacture of a medicament for the treatment of a cancer selected from: esophageal or head and neck squamous cell carcinoma; colorectal, ovarian, pancreatic or non-small cell lung cancer; and renal cell carcinoma.
In another embodiment, the cancer is selected from: esophageal or head and neck squamous cell carcinoma; colorectal, ovarian, pancreatic or non-small cell lung cancer; and and the immune checkpoint inhibitor is PDR001

### Pharmacology and Utility

Non-small cell lung cancer - In 2012, approximately 1.8 million people worldwide were diagnosed with lung cancer, and an estimated 1.6 million people died from the disease. Non-small cell lung cancer comprises approximately 85% of lung cancers, with adenocarcinomas and squamous cell carcinomas being the most common subtypes. Standard of care treatment for advanced stage non-small cell lung carcinomas (NSCLCs) that do not harbor genetic alterations in druggable driver oncogenes such as EGFR, ALK, or ROS includes chemotherapy and immunotherapy, administered concurrently or sequentially. While these treatments provide clinical benefit, the majority of patients experience disease progression within a year, and the prognosis for patients with advanced NSCLC remains poor. Immunotherapy for NSCLC with immune checkpoint inhibitors has demonstrated promise, with some NSCLC patients experiencing durable disease control for years. However, such long-term non-progressors are uncommon, and combination treatment strategies that can increase the proportion of patients responding to and achieving lasting remission with immunotherapy using checkpoint inhibitors are urgently needed. Activating mutations in the KRAS oncogene occur in approximately 30% of lung adenocarcinomas, and have been associated with poor outcome in some studies. No approved drugs target mutant KRAS directly, so standard of care for advanced stage KRAS-mutant NSCLC is also chemotherapy and immunotherapy as described above.

Head and neck squamous cell cancer - Squamous cell cancers are the most common cancers occurring in the head and neck, with an estimated worldwide incidence of approximately 686,000 for oropharyngeal and laryngeal cancers combined. Alcohol and tobacco use are the most common risk factors for head and neck squamous cell cancers (HNSCCs), with human papilloma virus (HPV) infection likely also playing a causative role. More than 90% of HNSCCs have overexpression of EGFR or its ligands. For patients with metastatic disease, standard systemic treatment includes platinum-based chemotherapy with or without cetuximab. Historically, median survival with systemic chemotherapy is approximately six months, with only approximately 20% of patients surviving one year. More recently, a survival benefit has been shown for nivolumab, an anti-programmed death-1 (PD-1) antibody, versus standard second-line single agent therapy (docetaxel, methotrexate, or cetuximab) in patients who had progressed on platinum-based chemotherapy. Still, the survival rate at one year for patients treated with nivolumab was only 36%. Therefore, a great need exists for improved treatments for this aggressive and debilitating cancer.

Colorectal cancer - Colorectal cancer (CRC) is the second most common cancer in women and the third most common cancer in men, accounting for an estimated 1.4 million new cancer cases worldwide in 2012. Chromosomal instability and microsatellite instability both play roles in the pathogenesis of CRC. Chromosomal instability is found in approximately 85% of sporadic colorectal cancers and is characterized by mutations in the Wnt pathway genes, APC and CTNNB1. KRAS mutations, occurring most commonly in codon 12 or 13, are present in approximately 45% of these cases and render anti-EGFR therapies ineffective. Microsatellite instability (MSI), arising due to defective DNA mismatch repair, is involved in approximately 15% of sporadic CRCs, as well as CRCs arising in Lynch syndrome due to a germline mutation of a mismatch repair gene. MSI-high CRCs tend to have a better prognosis than non-MSI-high CRC, and also have responded differently to some systemic therapies. Systemic therapy for metastatic CRC includes various agents used alone or in combination, including chemotherapies such as 5-Fluorouracil/leucovorin, capecitabine, oxaliplatin, and irinotecan; anti-angiogenic agents such as bevacizumab and ramucirumab; anti-EGFR agents including cetuximab and panitumumab for KRAS/NRAS wild-type cancers; and immunotherapies including nivolumab and pembrolizumab. Despite multiple active therapies, however, metastatic CRC remains incurable. While CRCs that are deficient in mismatch repair (MSI-high) exhibit high response rates to immune checkpoint inhibitor therapy, mismatch repair proficient CRCs do not. Since KRAS-mutant CRCs are typically mismatch repair proficient and are not candidates for anti-EGFR therapy, this subtype of CRC is particularly in need of improved therapies.

TNO155 is a first-in-class allosteric inhibitor of wild-type SHP2. SHP2 is a ubiquitously expressed non-receptor protein tyrosine phosphatase (PTP) composed of two N-terminal SH2 domains, a classic PTP domain, and a C-terminal tail. The phosphatase activity is auto-inhibited by the two SHP2 domains that bind to the PTP domain (closed conformation). Upon activation of receptor tyrosine kinases (RTKs), SHP2 is recruited to the plasma membrane where it associates with activated RTKs and a number of adaptor proteins to relay signaling by activating the RAS/MAPK pathway. TNO155 binds the inactive, or "closed" conformation of SHP2, thereby preventing its opening into the active conformation. This prevents the transduction of signaling from activated RTKs to the downstream RAS/MAPK pathway.

TNO155 has demonstrated efficacy in a wide range of RTK-dependent human cancer cell lines and in vivo xenografts. Preclinical *in vitro* and *in vivo* evaluation of TNO155 demonstrate selective and potent inhibition of the SHP2 phosphatase, in RTK-dependent human cancer models, for example, esophageal, HNSCC and NSCLC. SHP2 inhibition can be measured by assessing biomarkers within the MAPK signaling pathway, such as decreased levels of phosphorylated ERK1/2 (pERK) and downregulation of dual specificity phosphatase 6 (DUSP6) mRNA transcript. In the KYSE-520 (esophageal squamous cell carcinoma) and DETROIT-562 (pharyngeal squamous cell carcinoma) cancer cell lines, the *in vitro* pERK IC50's were 8 nM (3.4 ng/mL) and 35 nM (14.8 ng/mL) and the antiproliferation IC50's were 100 nM (42.2 ng/mL) and 470 nM (198.3 ng/mL), respectively. The antiproliferative effect of TNO155 was revealed to be most effective in cancer cell lines that are dependent on RTK signaling. *In vivo,* SHP2 inhibition by orally-administered TNO155 (20 mg/kg) achieved approximately 95% decrease in DUSP6 mRNA transcript in an EGFR-dependent DETROIT-562 cancer cell line and 47% regression when dosed on a twice-daily schedule. Dose fractionation studies, coupled with modulation of the tumor DUSP6 biomarker show that maximal efficacy is achieved when 50% PD inhibition is attained for at least 80% of the dosing interval.

In addition to its role in RAS-MAPK pathway activation downstream of RTKs, SHP2 is implicated in immune checkpoint and cytokine receptor signaling. In T cells, SHP2 was shown to be recruited by Programmed cell death-1 (PD-1) to dephosphorylate and inactivate the co-stimulatory receptor CD28, which suppresses T cell activation. SHP2 ablation in myeloid cells inhibited melanoma growth by potentiating production of the T-cell chemoattractant CXCL9 by macrophages in response to IFN-γ and tumor cell-derived cytokines, thereby facilitating tumor infiltration of IFN-γ-producing T cells. Therefore, inhibition of SHP2 may achieve anti-tumor efficacy through multiple mechanisms including direct inhibition of cancer cell growth, activation of tumor targeting T cells, and promotion of T cell tumor infiltration. SHP2, therefore, is implicated in PD-1 suppression of T-cell activation and the immune signaling in other immune-suppressive cells such as type 2 tumor associated macrophages (TAM).

The immune system is tightly controlled by a network of costimulatory and co-inhibitory ligands and receptors. These molecules provide the second signal for T cell activation and provide a balanced network of positive and negative signals to maximize immune responses against infection, while limiting immunity to self. Examples of costimulatory signals include the binding between the B7.1 (CD80) and B7.2 (CD86) ligands of the APC and the CD28 and CTLA-4 receptors of the CD4⁺ T-lymphocyte. Binding of B7.1 or B7.2 to CD28 stimulates T cell activation, whereas binding of B7.1 or B7.2 to CTLA-4 inhibits such activation. CD28 is constitutively expressed on the surface of T cells, whereas CTLA4 expression is rapidly up-regulated following T-cell activation. Other ligands of the CD28 receptor include a group of related B7 molecules, also known as the "B7 Superfamily". Several members of the B7 Superfamily are known, including B7.1 (CD80), B7.2 (CD86), the inducible co-stimulator ligand (ICOS-L), the programmed death-1 ligand (PD-L1; B7-H1), the programmed death-2 ligand (PD-L2; B7-DC), B7-H3, B7-H4 and B7-H6.

The Programmed Death 1 (PD-1) protein is an inhibitory member of the extended CD28/CTLA-4 family of T cell regulators. Two ligands for PD-1 have been identified, PD-L1 (B7-H1) and PD-L2 (B7-DC), that have been shown to downregulate T cell activation upon binding to PD-1. PD-L1 is abundant in a variety of human cancers.

PD-1 is known as an immunoinhibitory protein that negatively regulates TCR signals. The interaction between PD-1 and PD-L1 can act as an immune checkpoint, which can lead to, *e.g.,* a decrease in tumor infiltrating lymphocytes, a decrease in T-cell receptor mediated proliferation, and/or immune evasion by cancerous cells. Immune suppression can be reversed by inhibiting the local interaction of PD-1 with PD-L1 or PD-L2; the effect is additive when the interaction of PD-1 with PD-L2 is blocked as well.

PDR001 (spartalizumab) binds specifically and with high affinity to human PD-1. In Biacore assays, the constant of dissociation (KD) of spartalizumab on human PD-1 is 0.827 nM. In lymphocyte stimulation assays using human blood *ex vivo,* spartalizumab enhances IL-2 production by approximately 2-fold in response to super antigen stimulation with Staphylococcal enterotoxin B (SEB). Spartalizumab does not cross-react with rodent PD-1 and cannot be evaluated in murine tumor models. In order to evaluate pre-clinical activity of anti-PD1 therapy, a mouse antibody surrogate was developed (Clone 1D2) that demonstrates binding affinities to mouse PD1 antigen comparable to that of spartalizumab to human PD1 antigen. For further details, please refer to the PRD001 Investigator's Brochure. Combination efficacy and immune modulation by TNO155 with mouse anti-PD1 antibody in a syngeneic mouse tumor model, MC38, demonstrated a robust anti-tumor effect and significant inhibition of immune suppressive myeloid cells (gMDSC, TAMII) and lymphocytes (Treg cells) with an increase in activated CD8+ T-cells within the tumor. Collectively these data suggest that TNO155 in combination with an anti-PD1 therapy would provide an overall combination benefit because of enhanced anti-tumor immunity in addition to the tumor-intrinsic efficacy by inhibiting RAS-MAPK pathway activation.

SHP2 transduces signaling downstream of activated RTKs, as well as of PD-1 and CTLA4. Given the importance of immune checkpoint pathways in regulating an immune response, the need exists for developing novel combination therapies that activate the immune system. TNO155 is a potent inhibitor of wild-type SHP2, which enhances the anti-tumor activity of immune checkpoint inhibitors. Preclinical mouse syngeneic tumor model studies have demonstrated enhanced anti-tumor activity of an anti-PD-1 antibody when combined with TNO155. In addition, TNO155 has demonstrated single agent activity in preclinical NSCLC and HNSCC models, and anti-PD-1 agents have demonstrated clinical efficacy in subsets of NSCLC and HNSCC patients. A combination of the present invention, TNO155 and PDR001, shows improved efficacy compared to either single agent alone in the treatment of a syngeneic MC38 CRC tumor-bearing immune-competent mouse model.

The combinations disclosed herein can result in one or more of: an increase in antigen presentation, an increase in effector cell function (e.g., one or more of T cell proliferation, IFN-y secretion or cytolytic function), inhibition of regulatory T cell function, an effect on the activity of multiple cell types (e.g., regulatory T cell, effector T cells and NK cells), an increase in tumor infiltrating lymphocytes, an increase in T-cell receptor mediated proliferation, a decrease in immune evasion by cancerous cells, and a decrease in oncogenic activity (e.g., overexpression of an oncogene). In one embodiment, the use of a PD-1 inhibitor in the combinations inhibits, reduces or neutralizes one or more activities of PD-1, resulting in blockade or reduction of an immune checkpoint. Thus, such combinations can be used to treat or prevent disorders where enhancing an immune response in a subject is desired.

In certain embodiments, the cancer treated with the combination, includes but is not limited to, a solid tumor, a hematological cancer (e.g., leukemia, lymphoma, myeloma, e.g., multiple myeloma), and a metastatic lesion. In one embodiment, the cancer is a solid tumor. Examples of solid tumors include malignancies, e.g., sarcomas and carcinomas, e.g., adenocarcinomas of the various organ systems, such as those affecting the lung, breast, ovarian, lymphoid, gastrointestinal (e.g., colon), anal, genitals and genitourinary tract (e.g., renal, urothelial, bladder cells, prostate), pharynx, CNS (e.g., brain, neural or glial cells), head and neck, skin (e.g., melanoma), and pancreas, as well as adenocarcinomas which include malignancies such as colon cancers, rectal cancer, renal cancer (e.g., renal-cell carcinoma (clear cell or non-clear cell renal cell carcinoma), liver cancer, lung cancer (e.g., non-small cell lung cancer (squamous or non-squamous non-small cell lung cancer)), cancer of the small intestine and cancer of the esophagus. The cancer may be at an early, intermediate, late stage or metastatic cancer.

In some embodiments, the cancer is an advanced cancer. In some embodiments, the cancer is a metastatic cancer. In some embodiments, the cancer is a relapsed cancer. In some embodiments, the cancer is a refractory cancer. In some embodiments, the cancer is a recurrent cancer. In some embodiments, the cancer is an unresectable cancer.

In some embodiments, the cancer is a microsatellite instability-high (MSI-H) cancer. In some embodiments, the cancer is a mismatch repair deficient (dMMR) cancer.

In some embodiments, the cancer (e.g., cancer cells, cancer microenvironment, or both) has an elevated level of PD-L1 expression. Alternatively, or in combination, the cancer (e.g., cancer cells, cancer microenvironment, or both) can have increased IFNγ and/or CD8 expression.

In some embodiments, the subject has, or is identified as having, a cancer that has one or more of high PD-L1 level or expression, or as being tumor infiltrating lymphocyte (TIL)+ (e.g., as having an increased number of TILs), or both. In certain embodiments, the subject has, or is identified as having, a cancer that has high PD-L1 level or expression and that is TIL+. In some embodiments, the subject is identified based on having a cancer that has one or more of high PD-L1 level or expression, or as being TIL+, or both. In certain embodiments, the subject is identified based on having a cancer that has high PD-L1 level or expression and as being TIL+. In some embodiments, a cancer that is TIL+ is positive for CD8 and IFNγ. In some embodiments, the subject has, or is identified as having, a high percentage of cells that are positive for one, two or more of PD-L1, CD8, or IFNγ. In certain embodiments, the subject has, or is identified as having, a high percentage of cells that are positive for all of PD-L1, CD8, and IFNγ.

In some embodiments, the subject is identified based on having a high percentage of cells that are positive for one, two or more of PD-L1, CD8, and/or IFNγ. In certain embodiments, the subject is identified based on having a high percentage of cells that are positive for all of PD-L1, CD8, and IFNγ. In some embodiments, the subject has, or is identified as having, one, two or more of PD-L1, CD8, and/or IFNγ, and one or more of , esophageal cancer, an ovarian cancer, a breast cancer, a pancreatic cancer, a colorectal cancer, a skin cancer, a gastric cancer, an ER+ cancer, a head and neck squamous cell carcinoma, or a renal cell carcinoma. In certain embodiments, the subject is identified based on having one, two or more of PD-L1, CD8, and/or IFNγ, and one or more of a breast cancer, a pancreatic cancer, a colorectal cancer, a skin cancer, a gastric cancer, or an ER+ cancer).

Methods and compositions disclosed herein are useful for treating metastatic lesions associated with the aforementioned cancers.

The combinations as described herein can be administered to the subject systemically (e.g., orally, parenterally, subcutaneously, intravenously, rectally, intramuscularly, intraperitoneally, intranasally, transdermally, or by inhalation or intracavitary installation), topically, or by application to mucous membranes, such as the nose, throat and bronchial tubes.

Dosages and therapeutic regimens of the therapeutic agents disclosed herein can be determined. In some embodiments, the PD-1 inhibitor is administered by injection (e.g., subcutaneously or intravenously) at a dose (e.g., a flat dose) of about 100 mg to 600 mg, e.g., about 200 mg to 500 mg, e.g., about 250 mg to 450 mg, about 300 mg to 400 mg, about 250 mg to 350 mg, about 350 mg to 450 mg, or about 100 mg, about 200 mg, about 300 mg, or about 400 mg. The dosing schedule (e.g., flat dosing schedule) can vary from e.g., once a week to once every 2, 3, 4, 5, or 6 weeks. In one embodimen't, the PD-1 inhibitor is administered at a dose from about 300 mg to 400 mg once every three weeks or once every four weeks. In one embodiment, the PD-1 inhibitor is administered at a dose from about 300 mg once every three weeks. In one embodiment, the PD-1 inhibitor is administered at a dose from about 400 mg once every four weeks. In one embodiment, the PD-1 inhibitor is administered at a dose from about 300 mg once every four weeks. In one embodiment, the PD-1 inhibitor is administered at a dose from about 400 mg once every three weeks.

The epidermal growth factor receptor (EGFR) is an established critical therapeutic target in NSCLCs harboring activating EGFR mutations. Numerous trials with first (e.g. erlotinib, gefitinib) and second (e.g. afatinib, dacomitinib) generation EGFR inhibitors have been conducted in the EGFR-mutant advanced/unresectable NSCLC population, and have consistently demonstrated superior efficacy of EGFR tyrosine kinase inhibitors (TKIs) over chemotherapy in this population. Resistance to 1^{st} generation EGFR TKIs has been shown to arise through the development of an EGFR "gatekeeper" T790M mutation that impairs binding of the TKI, as well as by activation of alternative RTK pathways, including *MET* and *ERBB2* amplification. Clinical trials using 3^{rd} generation, irreversible EGFR inhibitors (e.g., osimertinib, rociletinib), which inhibit EGFR activating and gatekeeper mutations have demonstrated efficacy in EGFR T790M-mutant NSCLCs, highlighting their continued dependence on EGFR signaling. Emerging data from cancers that have become resistant to 3^{rd} generation inhibitors suggest that these cancers continue to select for activated RTK signaling, with resistance mutations in EGFR (C797S) as well as RTK amplifications (*MET*, *ERBB2, FGFR1*) having been described. Limited treatment options are available for patients whose cancers have developed resistance to 1^{st}/2^{nd} and 3^{rd} generation EGFR TKIs. Since SHP2 transduces EGFR signaling, and preclinical models have demonstrated a strong correlation between RTK dependence and SHP2 dependence, TNO155 is predicted to provide clinical benefit in these cancers whether resistance is driven by signaling from EGFR or another RTK.

More than 90% of head and neck cancers are characterized by overexpression or amplification of *EGFR*; amplification/overexpression of other RTKs, particularly FGFRs, and their ligands is also common. Inhibition of EGFR with cetuximab in advanced HNSCCs has also demonstrated clinical benefit, though disease control is not durable. The modest efficacy of EGFR inhibition in HNSCC may be related to compensatory signaling through other RTKs, which would be predicted to be abrogated by SHP2 inhibition with TNO155 treatment. In addition, preclinical testing identified head and neck cancer cells as the lineage with the highest frequency of sensitivity to SHP2 inhibition.

Patients with metastatic or unresectable RTK-driven cancers such as anaplastic lymphoma kinase (*ALK*)-rearranged NSCLC or stem cell factor receptor (*KIT*)-mutant gastrointestinal stromal tumor (GIST) derive benefit from molecules directly targeting these RTKs, but resistance to these agents invariably occurs. Mechanisms of resistance frequently include drug-resistant mutations in the targeted RTK and/or activation of bypass RTK pathways; in most cases, further treatment options are limited. Targeting SHP2 with TNO155 is a rational approach in such RTK-dependent cancers.

The data described herein, shows that inhibition of SHP2 with TNO155 at the MTD dose of 20 mg/kg BID, achieves anti-tumor efficacy as a single agent with significant combination benefit observed for TNO155 and anti-PD1 therapy. This combination also resulted in a decrease in subsets of suppressive immune populations such as T-regs, TAMII and gMDSCs. These data taken together demonstrate in a syngeneic mouse model that the combination of TNO155 and anti-PD1 therapy exerts anti-tumor activity, potentially as a consequence of decreased immunosuppressive myeloid cells and T-reg cells, and an increase in activated cytotoxic T-cells.

### Pharmaceutical Compositions

In another aspect, the present invention provides pharmaceutically acceptable compositions which comprise a therapeutically-effective amount TNO155 and a PD-1 inhibitor, formulated together with one or more pharmaceutically acceptable carriers (additives) and/or diluents. As described in detail below, the pharmaceutical compositions of the present invention may be specially formulated for administration in solid or liquid form, including those adapted for oral administration, for example, drenches (aqueous or non-aqueous solutions or suspensions), tablets, *e.g.,* those targeted for buccal, sublingual, and systemic absorption, boluses, powders, granules, pastes for application to the tongue.

The phrase "therapeutically-effective amount" as used herein means that amount of a compound, material, or composition comprising a compound of the present invention which is effective for producing some desired therapeutic effect in at least a sub-population of cells in an animal at a reasonable benefit/risk ratio applicable to any medical treatment.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, manufacturing aid (*e.g.,* lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

As set out above, certain embodiments of the present compounds may contain a basic functional group, such as amino or alkylamino, and are, thus, capable of forming pharmaceutically-acceptable salts with pharmaceutically-acceptable acids. The term "pharmaceutically-acceptable salts" in this respect, refers to the relatively non-toxic, inorganic and organic acid addition salts of compounds of the present invention. These salts can be prepared *in situ* in the administration vehicle or the dosage form manufacturing process, or by separately reacting a purified compound of the invention in its free base form with a suitable organic or inorganic acid, and isolating the salt thus formed during subsequent purification. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (See, for example, Berge et al. (1977) "Pharmaceutical Salts", J. Pharm. Sci. 66:1-19).

The pharmaceutically acceptable salts of the subject compounds include the conventional nontoxic salts or quaternary ammonium salts of the compounds, *e.g.,* from non-toxic organic or inorganic acids. For example, such conventional nontoxic salts include those derived from inorganic acids such as hydrochloride, hydrobromic, sulfuric, sulfamic, phosphoric, nitric, and the like; and the salts prepared from organic acids such as acetic, propionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like. The pharmaceutically acceptable salt of TNO155, for example, is succinate.

In other cases, the compounds of the present invention may contain one or more acidic functional groups and, thus, are capable of forming pharmaceutically-acceptable salts with pharmaceutically-acceptable bases. The term "pharmaceutically-acceptable salts" in these instances refers to the relatively non-toxic, inorganic and organic base addition salts of compounds of the present invention. These salts can likewise be prepared *in situ* in the administration vehicle or the dosage form manufacturing process, or by separately reacting the purified compound in its free acid form with a suitable base, such as the hydroxide, carbonate or bicarbonate of a pharmaceutically-acceptable metal cation, with ammonia, or with a pharmaceutically-acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like. (See, for example, Berge et al., *supra*)

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred per cent, this amount will range from about 0.1 per cent to about ninety-nine percent of active ingredient, preferably from about 5 per cent to about 70 per cent, most preferably from about 10 percent to about 30 percent.

In certain embodiments, a formulation of the present invention comprises an excipient selected from the group consisting of cyclodextrins, celluloses, liposomes, micelle forming agents, *e.g.,* bile acids, and polymeric carriers, *e.g.,* polyesters and polyanhydrides; and a compound of the present invention. In certain embodiments, an aforementioned formulation renders orally bioavailable a compound of the present invention.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution, suspension or solid dispersion in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules, trouches and the like), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds and surfactants, such as poloxamer and sodium lauryl sulfate; (7) wetting agents, such as, for example, cetyl alcohol, glycerol monostearate, and non-ionic surfactants; (8) absorbents, such as kaolin and bentonite clay; (9) lubricants, such as talc, calcium stearate, magnesium stearate, solid polyethylene glycols, sodium lauryl sulfate, zinc stearate, sodium stearate, stearic acid, and mixtures thereof; (10) coloring agents; and (11) controlled release agents such as crospovidone or ethyl cellulose. In the case of capsules, tablets and pills, the pharmaceutical compositions may also comprise buffering agents. Solid compositions of a similar type may also be employed as fillers in soft and hard-shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, *e.g.,* freeze-dried. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject compounds may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions.

When the compounds of the present invention are administered as pharmaceuticals, to humans and animals, they can be given per se or as a pharmaceutical composition containing, for example, 0.1 to 99% (more preferably, 10 to 30%) of active ingredient in combination with a pharmaceutically acceptable carrier.

The compounds of the present invention, which may be used in a suitable hydrated form, and/or the pharmaceutical compositions of the present invention, are formulated into pharmaceutically-acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of the combination of the invention will be that amount of each compound which is the lowest dose effective to produce a therapeutic effect. Such an effective dose will generally depend upon the factors described above.

In another aspect, the present invention provides pharmaceutically acceptable compositions which comprise a therapeutically-effective amount of one or more of the subject compounds, as described above, formulated together with one or more pharmaceutically acceptable carriers (additives) and/or diluents.

### Examples

### TNO155 and PDR001

(3S,4S)-8-(6-amino-5-((2-amino-3-chloropyridin-4-yl)thio)pyrazin-2-yl)-3-methyl-2-oxa-8-azaspiro[4.5]decan-4-amine (TNO155) is synthesized according to example 69 of WO2015/107495. PDR001 (spartalizumab) is detailed and can be made by vectors, host cells and methods described in US 2015/0210769, published on July 30, 2015, entitled "Antibody Molecules to PD-1 and Uses Thereof".

The utility of TNO155 and PDR001 described herein can be evidenced by testing in the following examples.

### Example 1

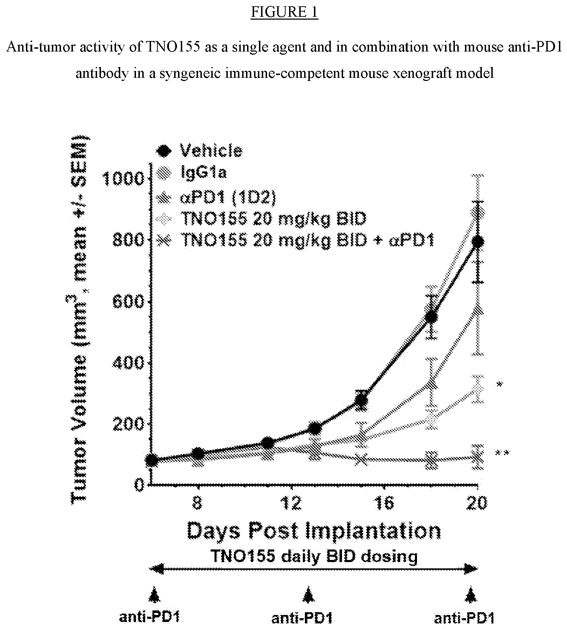

MC-38 tumors were established in female C57BL/6 mice by injection of one million cells in 50% Matrigel^{®} into the subcutaneous space of the right flank of each mouse (n=10/group). Six-days post-implantation test agents were administered at the dose levels and schedules indicated in figure 1. Tumor volumes of treatment groups vs. days post implantation are graphed. Statistical significance was calculated using Kruskal-Wallis One-way ANOVA and all-pairwise multiple comparison following Tukey's test on day 20 post implantation (p ≤ 0.05; p ≤ 0.01).

TNO155 was evaluated for single agent and combination anti-tumor activity with a mouse anti-PD1 antibody, Clone 1D2, in a syngeneic colorectal mouse model, MC38, implanted into immunocompromised NOD scid gamma (NSG) or immune-intact (C57BL/6) mice. MC38 cells harbor a PTPN11 G503V mutation that prevents inhibition of SHP2 by TNO155, thus allowing for direct interrogation of TNO155 immunomodulatory effects on tumors. TNO155 was evaluated in vitro in the MC38 cell line, in the absence of any immune cells, and demonstrated no effect on cell proliferation or viability. Similarly, a lack of anti-tumor activity was observed with TNO155, at 20 mg/kg twice a day (BID), on MC38 xenografts implanted into immunocompromised NSG mice. Due to the presence of the G503V mutation, TNO155 had no impact on downstream MAPK-signaling markers in tumor cells, such as phospho-ERK and phospho-RSK.

However, when the MC38 cell line was implanted into immune-competent C57BL/6 mice, and mice were treated with TNO155, 20 mg/kg BID daily, significant antitumor activity was observed (33.3% treatment/control (T/C), day 14 post-first dose). Treatment with the mouse anti-PD1 antibody, 10 mg/kg, once weekly (QW), also achieved significant anti-tumor growth inhibition (68.9% T/C, day 14 post-first dose). See Figure 1 showing that the combination of TNO155 (20 mg/kg BID) and anti-PD1 antibody (10 mg/kg, QW) displayed a significant improvement in efficacy (2.1%T/C, day 14 post-first dose) when compared to the single agent TNO155 and anti-PD1 antibody treatment groups.

### Example 2

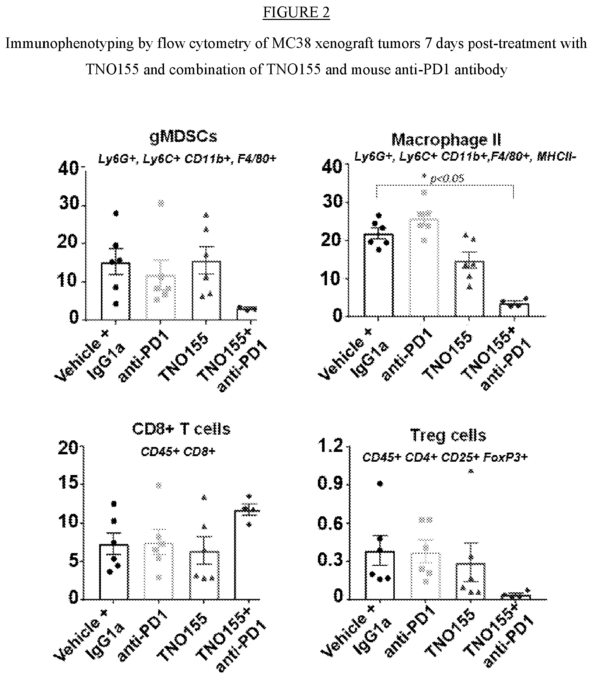

To further investigate the mechanism of efficacy in the MC38 model, immunophenotyping was performed on xenografts from all treatment groups at Day 7 of treatment. MC-38 tumors were established in female C57BL/6 mice by injection of one million cells in 50% Matrigel^{®} into the subcutaneous space of the right flank of each mouse (n=6/group). Seven-days post-implantation test agents were administered at: TNO155= 20 mg/kg BID; and mouse anti-PD1 (1D2 clone) = 10 mg/kg QW. Tumors were harvested, and were dissociated into single cells by enzymatic digestion. Cells were stained for markers associated with both lymphoid and myeloid phenotypes and analyzed by flow cytometry. Phenotypic markers are shown (see Figure 2) for two suppressive myeloid populations, granulocytic MDSCs and tumor associated macrophage type II in addition to the lymphoid populations of cytotoxic CD8+ T-cells and the suppressive regulator T-cells (Tregs). Shown is the percentage of the total CD45+ cells for the population ±SEM. One-way ANOVA pairwise comparison (p<0.05).

See figure 2. Flow cytometry analysis revealed that the combination of TNO155 and anti-PD1 displayed a robust decrease in the T-cell suppressive population of granulocytic myeloid derived suppressor cells (gMDSCs: Ly6G+, Ly6C+, C11b+, F4/80+), tumor associated macrophages type II (TAMII: Ly6G-, Ly6C+, CD11b+, F4/80+, MHCII-), and an increase in cytotoxic CD8+ T-cells (CD8+: CD45+, CD3+, CD8+) in the tumor. In line with the decrease in immune-suppressive immune cells, a significant decrease in the suppressive T-cell population (Treg: CD45+, CD4+, CD25+, FoxP3+) was also observed in the TNO155 and anti-PD1 combination group

These data demonstrate in a syngeneic mouse model that the combination of TNO155 and anti-PD1 therapy exerts anti-tumor activity as a consequence of decreased immunosuppressive myeloid cells and T-cells, and an increase in activated T-cells.

### Example 3

Investigation of the effect of TNO155 on M-CSF stimualted proliferation of CD14+ monocytes. Peripheral blood mononuclear cells (PBMC) were isolated from the whole blood of two donors (donor 1670 and E423) by CPT tubes and spinning at 1800 rpm for 20 minutes. CD14 positive monocytes were then isolated from PBMC using human Pan Monocyte Isolation Kit (Miltenyi Biotec # 130-096-537). 5000 monocytes suspended in 100 µL RPMI media were seeded in 96 well plates with 50 ng/mL recombinant M-CSF. 24 hours later, TNO155 was added at the indicated final concentrations. A CellTiter-Glo^{®} (CTG) cell viability assay was performed according to manufacturer's instructions using a separate plate with identically seeded cells and also performed for all plates after 6 days of incubation with TNO155. The Day 6 measurements were normalized with DMSO treated groups as 100% to evaluate the TNO155 effects on the M-CSF stimulated proliferation of monocytes and the Day 0 cell seeding measurement was indicated by the light dotted line.

This data shows that TNO155 can block the conversion of monocytes in the tumor microenvironment to immune-suppressive macrophages after being exposed to the M-CSF ligands in the tumors and synergizes with PD1 targeting agents that reinvigorate the exhausted T cells in the tumors.

The data presented provides *in vivo* evidence of SHP2's role in immune modulation in the tumor. The presence of the PTPN11-G503V mutation in the MC38, is an excellent tool to examine the effects of TNO155 specifically within the tumor microenvironment. Similar mutations in human SHP2, have been found to exist in JMML and Noonan syndrome patients where activating mutations, such as the G503V mutation, keep the SHP2 protein in an open confirmation and thus preventing the presence of the binding pocket for allosteric binding of TNO155. The presence of this mutation *in vivo,* in immunocompromised mice, results in a lack of efficacy and downstream MAPK pathway suppression, providing further evidence that this SHP2 mutation in the MC38 model prevents tumor intrinsic efficacy of TNO155. We show that inhibition of SHP2 with TNO155 at the MTD dose of 20 mg/kg BID, achieves anti-tumor efficacy as a single agent with significant combination benefit observed for TNO155 and anti-PD1 therapy. This combination also resulted in a decrease in subsets of suppressive immune populations such as T-regs, TAMII and gMDSCs. These data taken together demonstrate in a syngeneic mouse model that the combination of TNO155 and anti-PD1 therapy exerts anti-tumor activity, potentially as a consequence of decreased immunosuppressive myeloid cells and T-reg cells, and an increase in activated cytotoxic T-cells.

### Example 4

The initial regimen for TNO155 in combination with spartalizumab is based on data from the TNO155 first-in-human study, CTNO155X2101. Initially, TNO155 is dosed daily (QD) 2 weeks on/1 week off on a 21-day cycle (starting at 20 mg QD). Spartalizumab is dosed at 300 mg every 3 weeks on a 21-day cycle. In clinical trial protocol CTNO155B12101, patients with advanced EGFR WT, ALK WT, KRAS G12C and KRAS WT NSCLC are studied along with patients with advanced HNSCC (+/- naive to prior immuno-oncologic therapy).

Patients are treated with advanced solid tumors (with evaluable disease) fitting into one of the following groups: i). advanced EGFR WT, ALK WT NSCLC, after progression on or intolerance to platinum-containing combination chemotherapy; ii). Advanced HNSCC or esophageal SCC, after progression on or intolerance to platinum-containing combination therapy; iii). Advanced CRC, after progression on or intolerance to standard-of-care (SOC) therapy per local guidelines. Dose exapnsion treats patients with advanced solid tumors, with at least one measurable lesion, who fit into one of the following groups: i). advanced EGFR WT, ALK WT, KRAS G12C NSCLC with tumor PD-L1 ≥1%, after progression on or intolerance to platinum-containing combination chemotherapy and after progression on anti-PD-1 or anti-PD-L1 therapy; ii). advanced EGFR WT, ALK WT, KRAS WT NSCLC with tumor PD-L1 ≥1%, after progression on or intolerance to platinum-containing combination chemotherapy and after progression on anti-PD-1 or anti-PD-L1 therapy; iii). advanced HNSCC, after progression on or intolerance to platinum-containing combination chemotherapy.

A HNSCC patient, previously treated with radiotherapy in the adjuvant setting and, in the metastatic setting, received docetaxel, cisplatin, and fluorouracil, experienced a partial response (best response to chemotherapy lasting 1 month). Preliminary data from that same HNSCC patient treated with TNO155 20mg QD 2 weeks on/1 week off plus spartalizumab 300mg Q3W showed an unconfirmed partial response on the second tumor assessment (-30.6% decrease from baseline).

## Claims

1. A pharmaceutical combination comprising: (3S,4S)-8-(6-amino-5-((2-amino-3-chloropyridin-4-yl)thio)pyrazin-2-yl)-3-methyl-2-oxa-8-azaspiro[4.5]decan-4-amine (TNO155): 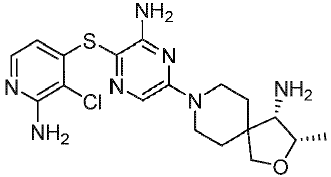 or pharmaceutically acceptable salt thereof,
and an immune checkpoint inhibitor.

2. (3S,4S)-8-(6-amino-5-((2-amino-3-chloropyridin-4-yl)thio)pyrazin-2-yl)-3-methyl-2-oxa-8-azaspiro[4.5]decan-4-amine (TNO155): 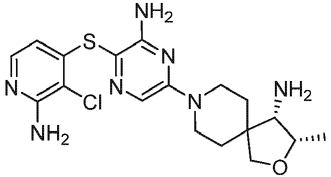 or a pharmaceutically acceptable salt thereof, for use in the treatment of cancer, wherein the treatment further comprises administration of an immune checkpoint inhibitor.

3. An immune checkpoint inhibitor for use in the treatment of cancer, wherein the treatment further comprises administration of (3S,4S)-8-(6-amino-5-((2-amino-3-chloropyridin-4-yl)thio)pyrazin-2-yl)-3-methyl-2-oxa-8-azaspiro[4.5]decan-4-amine (TNO155): 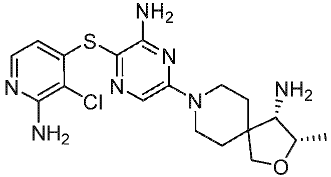 or a pharmaceutically acceptable salt thereof.

4. The pharmaceutical combination according to claim 1, the TNO155 or pharmaceutically acceptable salt thereof for use according to claim 2, or the immune checkpoint inhibitor for use according to claim 3, wherein the immune checkpoint inhibitor is a PD-1 inhibitor.

5. The pharmaceutical combination according to claim 4, the TNO155 or pharmaceutically acceptable salt thereof for use according to claim 4, or the immune checkpoint inhibitor for use according to claim 4, wherein the PD-1 inhibitor is PDR001, Nivolumab, Pembrolizumab, Pidilizumab, MEDI0680, REGN2810, TSR-042, PF-06801591, BGB-A317, BGB-108, INCSHR1210, or AMP-224.

6. The pharmaceutical combination according to any one of claims 1, 4 and 5, the TNO155 or pharmaceutically acceptable salt thereof for use according to any one of claims 2, 4 and 5, or the immune checkpoint inhibitor for use according to any one of claims 3 to 5, wherein the TNO155 or pharmaceutically acceptable salt thereof and the immune checkpoint inhibitor are in separate formulations.

7. The pharmaceutical combination according to any one of claims 1 and 4 to 6, for use as a medicament.

8. The pharmaceutical combination according to any one of claims 1 and 4 to 6, for use in the treatment of cancer.

9. The pharmaceutical combination for use according to claim 8, the TNO155 or pharmaceutically acceptable salt thereof for use according to any one of claims 2 and 4 to 6, or the immune checkpoint inhibitor for use according to any one of claims 3 to 6, wherein the cancer is selected from esophageal squamous cell carcinoma, head and neck squamous cell carcinoma, colorectal cancer, ovarian cancer, pancreatic cancer, non-small cell lung cancer and renal cell carcinoma.

10. The pharmaceutical combination for use according to any one of claims 7 to 9, the TNO155 or pharmaceutically acceptable salt thereof for use according to any one of claims 2, 4 to 6 and 9, or the immune checkpoint inhibitor for use according to any one of claims 3 to 6 and 9, wherein the TNO155 or pharmaceutically acceptable salt thereof and the immune checkpoint inhibitor are administered simultaneously, separately or over a period of time.

11. The pharmaceutical combination according for use according to any one of claims 7 to 10, the TNO155 or pharmaceutically acceptable salt thereof for use according to any one of claims 2, 4 to 6, 9 and 10, or the immune checkpoint inhibitor for use according to any one of claims 3 to 6, 9 and 10, wherein the TNO155 or pharmaceutically acceptable salt thereof is administered orally at a dose of about 1.5 mg per day, or 3 mg per day, or 6 mg per day, or 10 mg per day, or 20 mg per day, or 30 mg per day, or 40 mg per day, or 50 mg per day, or 60 mg per day, or 70 mg per day, or 80 mg per day, or 90 mg per day, or 100 mg per day.

12. The pharmaceutical combination for use according to any one of claims 7 to 11, the TNO155 or pharmaceutically acceptable salt thereof for use according to any one of claims 2, 4 to 6 and 9 to 11, or the immunue checkpoint inhibitor for use according to any one of claims 3 to 6 and 9 to 11, wherein the dose of TNO155per day is on a 21 day cycle of 2 weeks on drug followed by 1 week off drug.

13. The pharmaceutical combination for use according to any one of claims 7 to 12, the TNO155 or pharmaceutically acceptable salt thereof for use according to any one of claims 2, 4 to 6 and 9 to 12, or the immune checkpoint inhibitor for use according to any one of claims 3 to 6 and 9 to 12, wherein the immune checkpoint inhibitor is administered intravenously.

## Patentansprüche

1. Pharmazeutische Kombination, umfassend: (3S,4S)-8-(6-Amino-5-((2-amino-3-chlorpyridin-4-yl)thio)pyrazin-2-yl)-3-methyl-2-oxa-8-azaspiro[4.5]decan-4-amin (TNO155): 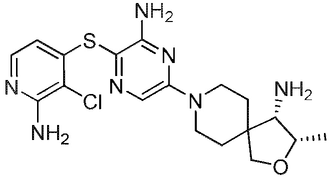 oder ein pharmazeutisch unbedenkliches Salz davon
und einen Immunkontrollpunkt-Inhibitor.

2. (3S,4S)-8-(6-Amino-5-((2-amino-3-chlorpyridin-4-yl)thio)pyrazin-2-yl)-3-methyl-2-oxa-8-azaspiro[4.5]decan-4-amin (TNO155): 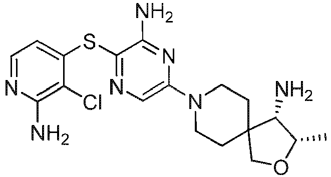 oder ein pharmazeutisch unbedenkliches Salz davon zur Verwendung bei der Behandlung von Krebs, wobei die Behandlung ferner die Verabreichung eines Immunkontrollpunkt-Inhibitors umfasst.

3. Immunkontrollpunkt-Inhibitor zur Verwendung bei der Behandlung von Krebs, wobei die Behandlung ferner die Verabreichung von (3S,4S)-8-(6-Amino-5-((2-amino-3-chlorpyridin-4-yl)thio)pyrazin-2-yl)-3-methyl-2-oxa-8-azaspiro[4.5]decan-4-amin (TNO155): 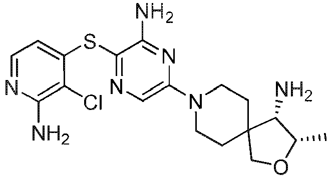 oder eines pharmazeutisch unbedenklichen Salzes davon umfasst.

4. Pharmazeutische Kombination nach Anspruch 1, TNO155 oder pharmazeutisch unbedenkliches Salz davon zur Verwendung nach Anspruch 2 oder Immunkontrollpunkt-Inhibitor zur Verwendung nach Anspruch 3, wobei es sich bei dem Immunkontrollpunkt-Inhibitor um einen PD-1-Inhibitor handelt.

5. Pharmazeutische Kombination nach Anspruch 4, TNO155 oder pharmazeutisch unbedenkliches Salz davon zur Verwendung nach Anspruch 4 oder Immunkontrollpunkt-Inhibitor zur Verwendung nach Anspruch 4, wobei es sich bei dem PD-1-Inhibitor um PDR001, Nivolumab, Pembrolizumab, Pidilizumab, MEDI0680, REGN2810, TSR-042, PF-06801591, BGB-A317, BGB-108, INCSHR1210 oder AMP-224 handelt.

6. Pharmazeutische Kombination nach einem der Ansprüche 1, 4 und 5, TNO155 oder pharmazeutisch unbedenkliches Salz davon zur Verwendung nach einem der Ansprüche 2, 4 und 5 oder Immunkontrollpunkt-Inhibitor zur Verwendung nach einem der Ansprüche 3 bis 5, wobei das TNO155 oder pharmazeutisch unbedenkliche Salz davon und der Immunkontrollpunkt-Inhibitor in separaten Formulierungen vorliegen.

7. Pharmazeutische Kombination nach einem der Ansprüche 1 und 4 bis 6 zur Verwendung als Arzneimittel.

8. Pharmazeutische Kombination nach einem der Ansprüche 1 und 4 bis 6 zur Verwendung bei der Behandlung von Krebs.

9. Pharmazeutische Kombination zur Verwendung nach Anspruch 8, TNO155 oder pharmazeutisch unbedenkliches Salz davon zur Verwendung nach einem der Ansprüche 2 und 4 bis 6 oder Immunkontrollpunkt-Inhibitor zur Verwendung nach einem der Ansprüche 3 bis 6, wobei der Krebs aus Speiseröhren-Plattenepithelkarzinom, Kopf-Hals-Plattenepithelkarzinom, Kolorektalkarzinom, Ovarialkarzinom, Bauchspeicheldrüsenkrebs, nichtkleinzelligem Lungenkrebs und Nierenzellkarzinom ausgewählt ist.

10. Pharmazeutische Kombination zur Verwendung nach einem der Ansprüche 7 bis 9, TNO155 oder pharmazeutisch unbedenkliches Salz davon zur Verwendung nach einem der Ansprüche 2, 4 bis 6 und 9 oder Immunkontrollpunkt-Inhibitor zur Verwendung nach einem der Ansprüche 3 bis 6 und 9, wobei das TNO155 oder pharmazeutisch unbedenkliche Salz davon und der Immunkontrollpunkt-Inhibitor gleichzeitig, getrennt oder über einen Zeitraum verabreicht werden.

11. Pharmazeutische Kombination gemäß zur Verwendung nach einem der Ansprüche 7 bis 10, TNO155 oder pharmazeutisch unbedenkliches Salz davon zur Verwendung nach einem der Ansprüche 2, 4 bis 6, 9 und 10 oder Immunkontrollpunkt-Inhibitor zur Verwendung nach einem der Ansprüche 3 bis 6, 9 und 10, wobei das TNO155 oder pharmazeutisch unbedenkliche Salz davon oral in einer Dosis von etwa 1,5 mg pro Tag oder 3 mg pro Tag oder 6 mg pro Tag oder 10 mg pro Tag oder 20 mg pro Tag oder 30 mg pro Tag oder 40 mg pro Tag oder 50 mg pro Tag oder 60 mg pro Tag oder 70 mg pro Tag oder 80 mg pro Tag oder 90 mg pro Tag oder 100 mg pro Tag verabreicht wird.

12. Pharmazeutische Kombination zur Verwendung nach einem der Ansprüche 7 bis 11, TNO155 oder pharmazeutisch unbedenkliches Salz davon zur Verwendung nach einem der Ansprüche 2, 4 bis 6 und 9 bis 11 oder Immunkontrollpunkt-Inhibitor zur Verwendung nach einem der Ansprüche 3 bis 6 und 9 bis 11, wobei die Tagesdosis von TNO155 einem 21-Tage-Zyklus von 2 Wochen Einnahme gefolgt von 1 Woche ohne Einnahme unterliegt.

13. Pharmazeutische Kombination zur Verwendung nach einem der Ansprüche 7 bis 12, TNO155 oder pharmazeutisch unbedenkliches Salz davon zur Verwendung nach einem der Ansprüche 2, 4 bis 6 und 9 bis 12 oder Immunkontrollpunkt-Inhibitor zur Verwendung nach einem der Ansprüche 3 bis 6 und 9 bis 12, wobei der Immunkontrollpunkt-Inhibitor intravenös verabreicht wird.

## Revendications

1. Combinaison pharmaceutique comprenant : (3S,4S)-8-(6-amino-5-((2-amino-3-chloropyridin-4-yl)thio)pyrazin-2-yl)-3-méthyl-2-oxa-8-azaspiro[4.5]décan-4-amine (TNO155) : 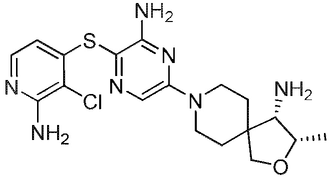 ou un sel pharmaceutiquement acceptable de celle-ci,
et un inhibiteur de point de contrôle immunitaire.

2. (3S,4S)-8-(6-amino-5-((2-amino-3-chloropyhdin-4-yl)thio)pyrazin-2-yl)-3-méthyl-2-oxa-8-azaspiro[4.5]décan-4-amine (TN0155) : 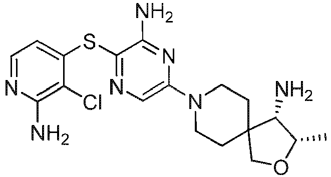 ou un sel pharmaceutiquement acceptable de celle-ci, pour une utilisation dans le traitement du cancer, dans laquelle le traitement comprend en outre l'administration d'un inhibiteur de point de contrôle immunitaire.

3. Inhibiteur de point de contrôle immunitaire pour une utilisation dans le traitement du cancer, dans lequel le traitement comprend en outre l'administration de (3S,4S)-8-(6-amino-5-((2-amino-3-chloropyridin-4-yl)thio)pyrazin-2-yl)-3-méthyl-2-oxa-8-azaspiro[4.5]décan-4-amine (TN0155) : 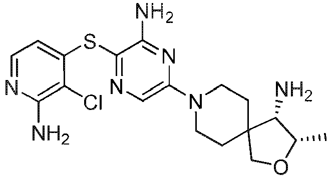 ou d'un sel pharmaceutiquement acceptable de celle-ci.

4. Combinaison pharmaceutique selon la revendication 1, TNO155 ou sel pharmaceutiquement acceptable de celle-ci pour une utilisation selon la revendication 2, ou inhibiteur de point de contrôle immunitaire pour une utilisation selon la revendication 3, dans laquelle l'inhibiteur de point de contrôle immunitaire est un inhibiteur de PD-1.

5. Combinaison pharmaceutique selon la revendication 4, TNO155 ou sel pharmaceutiquement acceptable de celle-ci pour une utilisation selon la revendication 4, ou inhibiteur de point de contrôle immunitaire pour une utilisation selon la revendication 4, dans laquelle l'inhibiteur de PD-1 est PDR001, Nivolumab, Pembrolizumab, Pidilizumab, MEDI0680, REGN2810, TSR-042, PF-06801591, BGB-A317, BGB-108, INCSHR1210, ou AMP-224.

6. Combinaison pharmaceutique selon l'une quelconque des revendications 1, 4 et 5, TNO155 ou sel pharmaceutiquement acceptable pour une utilisation selon l'une quelconque des revendications 2, 4 et 5, ou inhibiteur de point de contrôle immunitaire pour une utilisation selon l'une quelconque des revendications 3 à 5, dans laquelle le TNO155 ou le sel pharmaceutiquement acceptable et l'inhibiteur de point de contrôle immunitaire sont dans des formulations séparées.

7. Combinaison pharmaceutique selon l'une quelconque des revendications 1 et 4 à 6, pour une utilisation en tant que médicament.

8. Combinaison pharmaceutique selon l'une quelconque des revendications 1 et 4 à 6, pour une utilisation dans le traitement du cancer.

9. Combinaison pharmaceutique pour une utilisation selon la revendication 8, TNO155 ou sel pharmaceutiquement acceptable de celle-ci pour une utilisation selon l'une quelconque des revendications 2 et 4 à 6, ou inhibiteur de point de contrôle immunitaire pour une utilisation selon l'une quelconque des revendications 3 à 6, dans laquelle le cancer est choisi parmi le carcinome épidermoïde de l'œsophage, le carcinome épidermoïde de la tête et du cou, le cancer colorectal, le cancer de l'ovaire, le cancer du pancréas, le cancer du poumon non à petites cellules et le carcinome de cellules rénales.

10. Combinaison pharmaceutique pour une utilisation selon l'une quelconque des revendications 7 à 9, TNO155 ou sel pharmaceutiquement acceptable de celle-ci pour une utilisation selon l'une quelconque des revendications 2, 4 à 6 et 9, ou inhibiteur de point de contrôle immunitaire pour une utilisation selon l'une quelconque des revendications 3 à 6 et 9, dans laquelle le TNO155 ou le sel pharmaceutiquement acceptable et l'inhibiteur de point de contrôle immunitaire sont administrés simultanément, séparément ou sur une période de temps.

11. Combinaison pharmaceutique selon l'une quelconque des revendications 7 à 10, TNO155 ou sel pharmaceutiquement acceptable de celle-ci pour une utilisation selon l'une quelconque des revendications 2, 4 à 6, 9 et 10, ou inhibiteur de point de contrôle immunitaire pour une utilisation selon l'une quelconque des revendications 3 à 6, 9 et 10, dans laquelle le TNO155 ou le sel pharmaceutiquement acceptable de celle-ci est administré par voie orale à une dose d'environ 1,5 mg par jour, ou 3 mg par jour, ou 6 mg par jour, ou 10 mg par jour, ou 20 mg par jour, ou 30 mg par jour, ou 40 mg par jour, ou 50 mg par jour, ou 60 mg par jour, ou 70 mg par jour, ou 80 mg par jour, ou 90 mg par jour, ou 100 mg par jour.

12. Combinaison pharmaceutique pour une utilisation selon l'une quelconque des revendications 7 à 11, TNO155 ou sel pharmaceutiquement acceptable de celle-ci pour une utilisation selon l'une quelconque des revendications 2, 4 à 6 et 9 à 11, ou inhibiteur de point de contrôle immunitaire pour une utilisation selon l'une quelconque des revendications 3 à 6 et 9 à 11, dans laquelle la dose de TNO155 par jour est sur un cycle de 21 jours de 2 semaines sur un médicament suivi d'1 semaine sans médicament.

13. Combinaison pharmaceutique pour une utilisation selon l'une quelconque des revendications 7 à 12, TNO155 ou sel pharmaceutiquement acceptable de celle-ci pour une utilisation selon l'une quelconque des revendications 2, 4 à 6 et 9 à 12, ou inhibiteur de point de contrôle immunitaire pour une utilisation selon l'une quelconque des revendications 3 à 6 et 9 à 12, dans laquelle l'inhibiteur de point de contrôle immunitaire est administré par voie intraveineuse.
